# EUROPEAN PATENT SPECIFICATION

(11) **EP 3 549 095 B1**
(45) Date of publication and mention of the grant of the patent: **16.02.2022**
(21) Application number: 17877060.8
(22) Date of filing: 30.11.2017
(51) Int. Cl.: G06T 3/40

(54) **METHODS AND DEVICES FOR DISPLAYING IMAGE WITH CHANGED FIELD OF VIEW**
VERFAHREN UND VORRICHTUNG ZUR ANZEIGE VON BILDERN MIT VERÄNDERTEM SICHTFELD
PROCÉDÉS ET DISPOSITIFS D'AFFICHAGE D'UNE IMAGE AVEC UN CHAMP DE VISION MODIFIÉ

(30) Priority: 30.11.2016 US 201662427854 P
(43) Date of publication of application: 09.10.2019
(73) Proprietor: Novasight Ltd., 7019801 Airport City (IL)
(72) Inventor: YEHEZKEL, Oren, 5224154 Ramat Gan (IL); BELKIN, Michael, 5404402 Givat Shmuel (IL); YAM, Ran, 9682112 Jerusalem (IL); OZ, Dan, 4051418 Even Yehuda (IL)
(74) Representative: Harrison IP Limited
(86) International application number: PCT/IB2017/057516
(87) International publication number: WO 2018/100518

(56) References cited:
- WO-A1-2016/133886
- WO-A1-2016/149536
- CA-A1- 2 953 335
- US-A1- 2014 210 970
- US-A1- 2015 172 545
- US-A1- 2015 363 905
- US-A1- 2016 109 652

## Description

### RELATED APPLICATION

The present application gains priority from US provisional patent application 62/427,854 filed 30 November 2016.

### FIELD AND BACKGROUND OF THE INVENTION

The invention, in some embodiments, relates to the field of image display and, more particularly but not exclusively, to methods and devices for displaying an image of a scene on a display screen with a changed field of view. Some embodiments of the disclosed methods and devices are useful for increasing a human's visual perception of a scene, for example, in the field of ophthalmology for assisting a human having a deficient visual field.

Vision is an important sense with which a human perceives the world.

One aspect of vision is the visual field (herein, in some instances, such as in some instances in the priority document, used as a synonym for "field of view") the angular extent which a human visually perceives a scene at any one time.

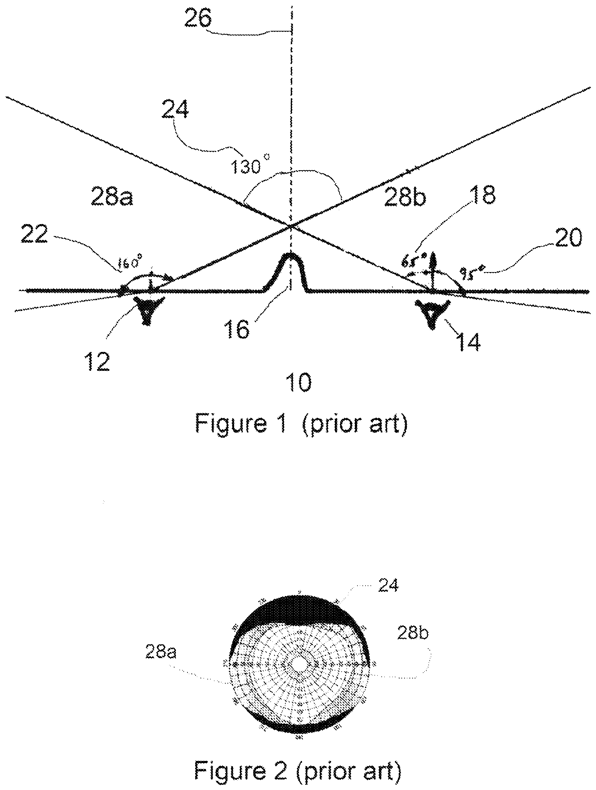

In healthy humans, the horizontal extent of the visual field of each one of the two eyes is ~65° nasally and ~95° temporally from the vertical meridian of the eye while the vertical extent of the visual field of each one of the two eyes is ~70° above and ~80° below the horizontal meridian of the eye. In Figure 1, a human head **10** is schematically depicted from above showing a left eye **12**, a right eye **14** and a nose **16.** The angular dimensions of the horizontal visual field of right eye **14** are indicated nasally **18** being 65° and temporally **20** being 95°, giving a total horizontal visual field **22** of 160° as indicated for left eye **12.**

Although the two eyes together give a human a visual field of ~190° (~95° temporally to either side), binocular vision is present only in the binocular portion **24** of the visual field where the visual fields of the two individual eyes **12** and **14** overlap. In healthy humans binocular visual field **24** is ∼130° horizontally (~65° to either side of the vertical meridian **26** that bisects the nose **16**). Only a single eye perceives the far peripheral visual field **28a** and **28b** of ~30° on either side of binocular visual field **24.**

A human has qualitatively different levels of perception in different parts of the visual field:
binocular central (foveal) visual field, a ∼2° diameter circle centered in the binocular visual field **24**;
binocular macular visual field, a ~20° diameter oval centered in the binocular visual field **24**;
binocular near peripheral visual field, 30° to either side of the vertical meridian **26** that bisects the nose **16**;
binocular mid peripheral visual field, 60° to either side of the vertical meridian **26** that bisects the nose **16**; and
monocular far peripheral portion **28a** and **28b** of 30° on either side of the binocular portion of the visual field **24.**

In Figure 2 is depicted a standard-format graphic representations of the angular dimensions of the two-eye visual field both vertical and horizontal of a normal human (NASA SP-3006, roughly reproduced from an image found in the Bioastronautics Data Book, 1964).

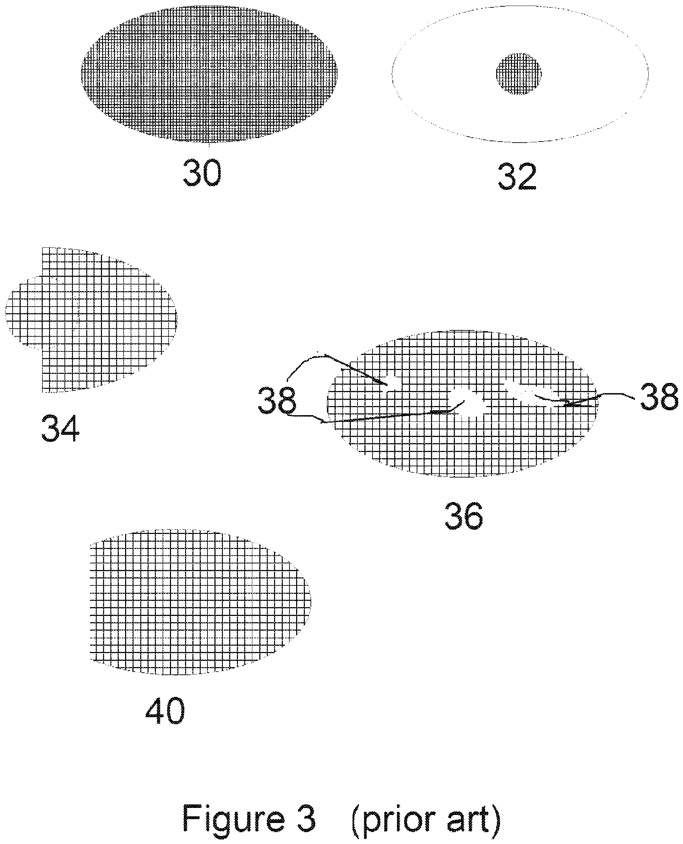

In some humans, visual field deficiency occurs for various reasons, e.g., physical damage to one or both of the eyes or to the brain. Some visual field deficiences include:
- tunnel vision (the angular dimensions of the visual field are limited to more central portions of a normal visual field) that can be caused, *inter alia*, by glaucoma and retinitis pigmentosa but also occurs when a human has a normal visual field but wears a device such as eyeglasses, masks, goggles or a helmet (in Figure 3, a representation of the angular dimensions of the two-eye visual field of a normal human **30**, and **32** of a human suffering from tunnel vision having a 20° visual field corresponding to intact macular and foveal visual fields but a complete loss of peripheral visual fields);
- hemianopsia where the macular and foveal visual fields are intact with loss of half of the peripheral visual fields (in Figure 3, 34 a representation of the angular dimensions of the two-eye visual field of a human suffering from left hemianopsia with intact macular and foveal visual fields but with loss of peripheral vision to the left of the vertical meridian of the head);
- scotoma where the visual field includes one or more localized blind spots (in Figure 3, 36 a representation of the angular dimensions of the two-eye visual field of a human suffering from scotoma **38**, also affecting the foveal and macular visual fields; and
- single eye vision in Figure 3, 40 a representation of the angular dimensions of a one-eye visual field of a human.

Various methods have been proposed for assisting humans having visual fields deficiency, see for example PCT patent publication WO 2016/103259, US Patent Publication US 2016-015685 and Zhu Y, Chang J, Niu J, Chen W L and Du X Y in Optics Express 2016 24(2), p.1305. Some known methods include using lenses and prisms to optically direct an image having a relatively large field of view into portions of the visual field of a human that are still intact. Documents US 2014/210970 A1 and WO 2016/149536 A1 are also relevant to the present invention.

### SUMMARY OF THE INVENTION

Some embodiments of the invention herein relate to methods and devices for displaying an image of a scene on a display screen with a changed field of view. Some embodiments of the disclosed methods and devices are useful for artificially increasing the effective visual field of a human, for example when such a human has a visual field deficiency.

Some embodiments of the invention include acquiring a base image of a scene with a base field of view and then displaying the entire scene with a display image that has a display field of view that is smaller than the base field of view. In some embodiments, the size of the display field of view corresponds to the size of the actual visual field of one or both eyes of a human so that the invention thereby artificially increases the effective visual field of the human by "compressing" the base image of the scene having the larger base field of view to a display image having the smaller display field of view.

According to an aspect of some embodiments of the present invention there is provided an image-display method comprising:
a1. positioning a first display screen so that a display surface of the first display screen fills substantially the entire visual field of a first eye of a human and is not visible to a second eye of the human;
b1. at a first display refresh-rate:
   i. from a first video stream extracting a pixelated still first base image having a first base field of view, the first base image representing a first scene, the first base image being digital image data stored in a digital memory;
   ii. from the first base image, creating a pixelated first display image being digital image data representing the entire first scene, the first display image having a first display field of view different from the first base field of view, so that the first scene as represented by the first display image has a field of view not greater than the visual field of the first eye, the creating the first display image comprising while retaining the position of each pixel relative to neighboring pixels translating pixels, each translated pixel from a base coordinate in the first base image to a display coordinate in the first display image, and
   iii. on the first display screen displaying the first display image to the first eye so that the entire the first field of view of the first display image is perceived by the visual field of the first eye at one time,
   thereby allowing the first eye to perceive an entirety of the first scene at one time. the method further comprises: determining the gaze direction of the first eye; and creating and/or displaying the first display image also based on the determined gaze direction.

In some embodiments, during the creating of the first display image, pixels from a specific portion of the first base image are preserved and not translated so that the preserved portion of the first display image corresponding to the preserved portion of the first base image are the same. In some such embodiments, the method further comprises: determining the gaze direction of the first eye; and creating and/or displaying the first display image also based on the determined gaze direction so that the preserved portions of the first base image that correspond to specific portions of the visual field of the first eye are perceived by the specific portions of the visual field of the first eye in the first display image.

In some embodiments, during the creating of the first display image, pixels from portions of the first base image corresponding to at least part of the foveal visual field of the first eye are not translated so that the portions of the first display image corresponding to the part of the foveal visual field of the first eye are the same as the corresponding portion of the first base image. In some such embodiments, the method further comprises: determining the gaze direction of the first eye; and creating and/or displaying the first display image also in accordance with the determined gaze direction so that the portion of the first display image corresponding to the part of the foveal visual field of the first eye is positioned (on the first display screen) to be perceived by the foveal visual field of the first eye.

In some embodiments, the visual field of the first eye includes at least one blind spot and at least some of the translation of the pixels is such that substantially no pixels representing the scene are located at a portion of the first display image that corresponds to at least one of the at least one blind spot, the method further comprising: determining the gaze direction of the first eye; and displaying and/or creating the first display image also in accordance with the determined gaze direction so that substantially no pixels representing the scene are located at a portion of the first display image that corresponds to at least one of the at least one blind spots in the visual field of the eye.

In some embodiments, the base field of view has at least one angular dimension greater than the visual field of the first eye; and the translation of pixels for the creating of the first display image is such that the greater angular dimension of the scene is compressed into the display field of view of the first display image.

In some embodiments, no display screen is positioned before the second eye of the human.

In some embodiments, the method further comprises:
a2. positioning a second display screen so that a display surface of the second display screen fills substantially the entire visual field of a second eye of a human and is not visible to the first eye of the human;
b2. at a second display refresh-rate:
   i. from a second video stream extracting a pixelated still second base image having a second base field of view, the second base image representing a second scene, the second base image being digital image data stored in a digital memory,
   ii. from the second image, creating a pixelated second display image being digtal image data representing the entire second scene, the second display image having a second display field of view different from the second base field of view, so that the second scene as represented by the second display image has a field of view not greater than the visual field of the second eye, the creating the second display image comprising while retaining the position of each pixel relative to neighboring pixels translating pixels, each translated pixel from a base coordinate in the second base image to a display coordinate in the second display image, and
   iii. on the second display screen displaying the second display image to the second eye so that the entire the second field of view of the second display image is perceived by the visual field of the second eye at one time,
   thereby allowing the second eye to perceive an entirety of the second scene at one time.

According to an aspect of some embodiments of the present invention there is also provided a monocular headset configured to be worn on the head of a human, comprising:
a single display screen mounted on a headset body, so that when the headset body is worn on the head of a human, the display screen is positioned so that a display surface of the display screen fills substantially the entire visual field of a first eye of a human and is not visible to a second eye of the human; and
functionally associated with the display screen, a digital processor including a video input port configured to accept a video stream via the video input port and to implement an embodiment of a method according to the teachings herein, with the video stream using the display screen, optionally further comprising an eye-tracker to determine the gaze direction of the first eye and to provide the determined gaze direction to the processor. In some embodiments, the headset further comprises a digital video camera with a video outlet port functionally associated with the video input port of the processor, the video camera configured to acquire video images and to output a digital video stream corresponding to the video images via the video outlet port to the processor.

According to an aspect of some embodiments of the present invention there is also provided a binocular headset configured to be worn on the head of a human, comprising:
a first display screen mounted on a headset body, so that when the headset body is worn on the head of a human, the first display screen is positioned so that a display surface of the first display screen fills substantially the entire visual field of a first eye of a human and is not visible to a second eye of the human;
a second display screen mounted on the headset body, so that when the headset body is worn on the head of a human, the second display screen is positioned so that a display surface of the second display screen fills substantially the entire visual field of a second eye of a human and is not visible to the first eye of the human; and
functionally associated with the first display screen and with the second display screen, a digital processor including a video input port configured to accept a video stream via the video input port and to implement an embodiment of a method according to the teachings herein with the video stream using the first display screen and the second display screen,, optionally further comprising an eye-tracker to determine the gaze direction of the first eye and of the second eye, and to provide the determined gaze directions to the processor. In some embodiments the headset firther comprises at least one of:
   i. a digital binocular video camera with a video outlet port functionally associated with the video input port of the processor, the binocular video camera configured to acquire binocular pairs of monocular video images and to output a digital binocular video stream corresponding to the video images via the video outlet port to the processor; and
   ii. a first digital video camera with a first video outlet port functionally associated with the video input port of the processor, the first video camera configured to acquire monocular video images and to output a first monocular digital video stream corresponding to the monocular video images via the video outlet port to the processor, and a second digital video camera with a second video outlet port functionally associated with the video input port of the processor, the second video camera configured to acquire monocular video images and to output a second monocular digital video stream corresponding to the monocular video images via the video outlet port to the processor.

### BRIEF DESCRIPTION OF THE FIGURES

Some embodiments of the invention are described herein with reference to the accompanying figures. The description, together with the figures, makes apparent to a person having ordinary skill in the art how some embodiments of the invention may be practiced. The figures are for the purpose of illustrative discussion and no attempt is made to show structural details of an embodiment in more detail than is necessary for a fundamental understanding of the invention. For the sake of clarity, some objects depicted in the figures are not to scale.

In the Figures:
Fig. 1 (prior art) is a schematic depictions of a human head from above showing aspects of the horizontal visual fields of a normal human;
Fig. 2 (prior art) depicts a standard-format graphic representations of the angular dimensions of the visual field (both vertical and horizontal) of a normal human;
Fig. 3 (prior art) depicts representations of visual fields of a normal human **30**, a human suffering from tunnel vsion **32**, a human suffering from hemianopsia **34.** a human suffering from scotoma **36** and a human having single eye vision;
Fig. 4 is an explanatory graph qualitatively showing the magnitude of radial translation of pixels in a base image to create a display image as a function of distance from a point, to graphically depict inhomogeneous and smooth translation of pixels in accordance with some embodiments of the teachings herein;
Fig. 5 is a schematic depiction of the creation of a display image from a base image for a human suffering from single-eye vision according to an embodiment of the teachings herein;
Fig. 6 is a schematic depiction of creation of a first and second display image from a first and second base image for a human suffering from hemianopsia according to an embodiment of the teachings herein;
Fig. 7 is a schematic depiction of creation of a first display image from a first base image to increase the visual field of the human according to an embodiment of the teachings herein;
Figs. 8A and 8B are schematic depictions of a monocular headset according to an embodiment of the teachings herein;
Figs. 9A and 9B are schematic depictions of a binocular headset according to an embodiment of the teachings herein; and
Fig. 10 is a schematic depictions of a binocular headset according to an embodiment of the teachings herein.

### DESCRIPTION OF SOME EMBODIMENTS OF THE INVENTION

Some embodiments of the invention herein relate to methods and devices for displaying an image of a scene on a display screen with a changed field of view. Some embodiments of the disclosed methods and devices are useful for artificially increasing the effective visual field of a human, for example when such a human has a field of view deficiency.

### Image display method according to the teachings herein

Some embodiments of the teachings herein relate to methods for displaying an image with a changed field of view.

Some embodiments of the methods according to the teachings herein are useful to increase the spatial awareness of a human, including a human having no visual field deficiency. As noted above, some implementation of the teachings herein artificially increases the effective visual field of the human by "compressing" an image of a scene with large angular dimensions into the smaller visual field of the human. Such embodiments can be useful for pilots (of manned or unmanned aircraft) or operators of remote devices to increase the actual visual field perceived, for example, to perceive motion that is 150° temporally from the vertical meridian of the head.

Some embodiments of the methods according to the teachings herein are useful to increase the spatial awareness of a human having a visual field deficiency. Implementation of the teachings "compresses" an image of a scene (for example, a scene that has angular dimensions that are entirely perceived by a human having no visual field deficiency) into the actual visual field of the human. For example, for a human having tunnel vision with a horizontal visual field of 90° (±45° from the vertical meridian of the head), an image of a scene having a horizontal dimension of 120° is "compressed" into the extant visual field, increasing the human's perception of their environment.

In some preferred embodiments, "compression" is not performed for the part image that corresponds to some or all of the foveal visual field as it is generally preferable to preserve maximal foveal vision (e.g., in terms of resolution, actuity). In some preferred embodiments, "compression" is not performed for the part image that corresponds to some or all of the macular visual field.

Thus, according to an aspect of some embodiments of the teachings herein there is provided an image-display method according to appended claim 1. thereby allowing the first eye to perceive an entirety of the first scene at one time.

In instances where translation of neighboring pixels in a base image brings the pixels too close together for the desired (or possible) display image resolution, the pixels are united in the usual way for resizing of a graphic image as known in the art of digital graphic display.

### First display screen

The first display screen is any suitable display screen. Due to availability and well-defined characteristics, in some preferred embodiments the first display screen is of the type and technology known in the art of virtual reality (VR) as implemented in VR headsets, e.g., LCD or OLED. In some preferred embodiments, the first display screen is a color screen.

In some embodiments, the first display screen is positioned so that the first eye directly views the display surface of the first screen, as is known in the art of VR. In some embodiments, the first display screen is positioned so that the first eye directly views the display surface of the first screen through an intervening optical component, e.g., a filter or lens: some such embodiments allow easily modifying a field of view of a standard display screen as required for use by a specific human. In some embodiments, the display screen is a virtual retinal display (i.e., retinal projector) that actively projects a desired display image onto the retina of a human eye.

Higher display-screen pixel density is preferred to lower pixel density to provide a better display image. In some embodiments, the pixel density is greater than 400 ppi, greater than 500 ppi, greater than 600 ppi, greater than 700 ppi and even greater than 800 ppi (e.g., based on HDS IPS LCD technology such as Triluminos^{™} by Sony Corporation (Minato, Tokyo, Japan). Preferably, the pixel density is pixel density at least as high as known to be acceptable in the art of VR.

As noted above, the first display screen is positioned so that a display surface of the first display screen fills substantially the entire visual field of the first eye and is not visible to the second eye of the human. This positioning may be performed in any suitable way, typically by positioning the first display screen in front of the first eye. In some preferred embodiments, the first display screen is -mounted so that the position relative to the first eye remains constant even when the head of the human moves. In some preferred embodiments, such positioning and mounting is implemented by mounting the first display screen in a headset with a display screen which, when worn by the human, leads to the proper positioning of the first display screen in front of the eye in a way that allows the display surface of the first display screen to fill substantially the entire visual field of the first eye and not to be visible to the second eye of the human. Such headsets are well-known in the field of virtual reality headsets. Some preferred embodiments of the teachings herein are implemented using commercially-available VR headsets such as HTC Vive^{™} by HTC corporation (Xindian District, New Taipei City, Taiwan), Oculus Rift^{™} by Oculus VR (Menlo Park, California, USA), Play Station VR by Sony Corporation (Minato, Tokyo, Japan), FOVE^{™} by Fove Inc. (San Mateo, California, USA) and Gear VR^{™} and ExynosVR^{™} by Samsung Group (Seoul, South Korea). In some embodiments, such commmercially available headsets are hardware, firmware or software modified to implement the teachings herein.

The display screen refresh rate is any suitable refresh rate. As is known in the art of VR, a minimal acceptable refresh rate is not less than 60Hz, but higher refresh rates are preferred. Accordingly, in some embodiments the display refresh rate is not slower than 60 Hz, not slower than 90 Hz and even not slower than 120 Hz.

As noted above, the first display screen is positioned so that the display surface of the first display screen fills substantially the entire visual field of the first eye. Although the term "...fills substantially the entire visual field of the first eye..." is clear to a person having ordinary skill in the art, embodiments of the term are further clarified hereinbelow.

Ideally, the first display screen is positioned so that the display surface literally fills the entire visual field of the first eye, including when the eye moves relative to the head, up, down, nasally or temporally. Such an ideal situation is relatively easily implemented when the human for which the teachings herein are implemented has tunnel vision or a similar visual field deficiency.

Challenges to literally filling the entire visual field at the desired resolution, especially when implementing the teachings herein for a human having no substantial visual field deficiency include: the required display screen may be too large, too expensive, not readily available, or require too much processing power and electrical power to be practical.

Accordingly, in some embodiments, the first display screen is positioned such that the display surface fills the entire visual field of the first eye when the first eye gazes straight ahead: in such embodiments when the first eye gazes up, down, nasally or temporally it is possible that some part of the visual field of the first eye sees something that is not the display surface of the first display screen.

Accordingly, in some embodiments, the first display screen is positioned such that the display surface fills the entire mid-peripheral visual field of the first eye when the first eye gazes straight ahead: in such embodiments when the first eye gazes up, down, nasally or temporally it is possible that some part of the visual field of the first eye sees something that is not the display surface of the first display screen. In some such embodiments, the term "...fills substantially the entire visual field of a first eye of a human first eye..." as used herein means that the first display screen is positioned such that relative to the first eye gazing straight ahead, the first display screen fills the visual field of the first eye horizontally not less than 60° nasally, not less than 60° temporally, vertically not less than 70° up and vertically not less than 80° down.

To increase the general availability of the teachings herein, in some embodiments it is preferred to use a commercially-available devices for implementing the teachings herein. As is known to a person having ordinary skill in the art of VR, at the time of this writing typical high-end VR headsets provide two display screens configured and positioned so that the display surface of each one of the two display screens (one for the left eye, one for the right eye) fills substantially the entire mid-peripheral visual field of each eye when the eye gazes straight ahead: horizontally 60° nasally and 50° temporally, vertically up and down both 50°. Accordingly, in some embodiments, the term "...fills substantially the entire visual field of a first eye of a human first eye..." as used herein means that the first display screen is positioned such that relative to the first eye gazing straight ahead, the first display screen fills the visual field of the first eye horizontally not less than 50° nasally, not less than 40° temporally, vertically not less than 40° up and vertically not less than 40° down. Accordingly, in some embodiments, the term "...fills substantially the entire visual field of a first eye of a human first eye..." as used herein means that the first display screen is positioned such that relative to the first eye gazing straight ahead, the first display screen fills the visual field of the first eye horizontally not less than 60° nasally, not less than 50° temporally, vertically not less than 50° up and vertically not less than 50° down.

### Base Image

As noted above, from a video stream a pixelated still base image (the first base image) having a base field of view is extracted, where the first base image represents a scene having angular dimensions.

The first base image (as well as the first display image and the second base image and second display image, vide infra) is digital image data stored in a digital memory (for example, on magnetic or electrical media, e.g., computer memory, flash memory, solid state memory, magnetic media, random-access memory (RAM), including memory (e.g., RAM memory of a graphic processing unit) which digital image data can be visually displayed to a human using known electronic image display devices such as a VR display screen.

The scene is the visual scene which it is desired that the human visually perceives. The first base image is a still image of the scene that is extracted from the video stream (e.g., a single frame). The scene has angular dimensions (i.e., the field of view of the scene).

### Video stream

The video stream is any suitable video stream.

In some embodiments, the video stream is a prerecorded video stream.

In some embodiments, the video stream is a video stream acquired concurrently with implementation of the method in real time. Accordingly, in some such embodiments, the first base image is provided in real time from a concurrently-acquired video stream.

In some embodiments, the video stream is acquired with a remote video camera, that is to say, a video camera that is not carried by the human.

In some embodiments, the remote video camera is mounted on a remotely-operated device, for example a remotely operated device selected from the group of a robot, a vehicle, a drone and a UAV.

In some preferred embodiments, the video stream is acquired with a video camera mounted on the head of the human, e.g., a video camera mounted on a VR headset of which components are used to implement the method. In some embodiments, the video stream is acquired with a video camera mounted on the head of the human, immediately in front of the first eye. e.g., a video camera mounted on the front of a VR headset directly in front of the first eye.

Any suitable video camera may be used for implementing the teachings herein, for example, a video camera as is known in the art of smartphones.

The video stream has any suitable frame rate. In some embodiments, the video stream has a frame rate that is identical to the display refresh rate. In some embodiments, the video stream has a frame rate that is greater that the display refresh rate and the method further comprises extracting a first base image at the display refresh rate. In some embodiments, the video stream has a frame rate that is slower that the display refresh rate and the method further comprises extracting a first base image at the display refresh rate by optionally synthesizing an in-between frame as well-known in the art, e.g., as implemented in the PlayStation VR

The video stream has any suitable field of view, typically having the same or larger angular dimensions than of the scene. In some embodiments, the video stream has a field of view substantially identical to the base field of view of the first base image. In some embodiments, the video stream has a field of view greater than the base field of view of the first base image.

### Creating and displaying a first display image

As noted above, from the first base image, a pixelated first display image (digital image data representing the entire scene) is created.

Creating the first display image comprises translating pixels each from a base coordinate in the first base image (where the pixel is found in the first base image) to a display coordinate in the display image (where the same pixel is found in the first display image) while retaining the position of each pixel relative to neighboring pixels. The first display image can thus be considered to be a distorted version of the first base image. Methods for performing such distortion are well-known in the field of image processing and video gaming and can be performed, for example using methods implemented in commercially-available software such as Morpheus Software (Santa Barbara, California, USA).

The first base image has a base field of view that is, for some reason, not acceptable for display to the first eye as-is (e.g., due to a visual field deficiency of the first eye), typically because the first eye is unable to perceive the entirety of the scene as represented by the first base image (if the first base image were to be displayed) at one time. The distortion performed is such that the first display image has a display field of view that is different from the base field of view of the first base image. The difference between the display field of view and the base field of view is such that when the first display image is displayed on the first display screen, the entire field of view of the first display image is perceived by the visual field of the first eye at one time, thereby allowing the first eye to perceive the entirety of the scene at one time.

It is important to note that in some embodiments where the method is implemented to display an image to a human having a deficient visual field, the visual field of the human is first mapped and the map is used as a guide for creating the display image, specifically, as which pixels in the base image to translate to where in the display image.

### Smooth Translation

In preferred embodiments, during creation of a first display image from a first base image, all of the translations of pixels are smooth, that is to say, there are no discontinuities in the first display image. More specifically, a function describing the translation of a series of pixels (e.g., pixels along a line of pixels) is a smooth function that has no discontinuous derivatives.

### Eye tracking

As known in the art of VR, one preferred method to improve the VR experience is by determining the gaze direction of each one of the two eyes and, based on the gaze direction of each eye, creating and/or displaying the images to be displayed to the eyes. Accordingly, in some embodiments the method further comprises: determining the gaze direction of the first eye (e.g., using an eye tracker as known in the art); and creating and/or displaying the first display image also based one the determined gaze direction.

### Preserved image portions

In some embodiments, not all portions of a first base image are distorted to create the first display image so that these preserved portions are the same in the first base image and the first display image. Accordingly, in some embodiments, during the creating of the first display image, pixels from a specific portion of the first base image are preserved and not translated so that the respective preserved portion of the first display image corresponding to the preserved portions of the first base image are the same.

In some preferred embodiments, the method further comprises: determining the gaze direction of the first eye; and creating and/or displaying the first display image also based on the determined gaze direction so that the preserved portions of the first base image that correspond to specific portions of the visual field of the first eye are perceived by the specific portions of the visual field of the first eye in the first display image.

### Foveal visual field

The foveal visual field is a very small (~2° diameter) circle centered in binocular visual field of an eye that provides the particularly sharp central vision called foveal vision which is necessary for activities such as reading or recognizing faces.

As known in the art of VR, one preferred method to improve the VR experience, to save computing power and to increase the attainable frame rate is by using foveated rendering. In foveated rendering the gaze direction of each of the two eyes is continuously determined. Based on the gaze direction of each eye, the portion of an image that is perceived by the fovea is displayed at a higher resolution (preferably equal to or higher than the foveal resolution) while other portions of the image are displayed at a lower resolution (preferably equal to or higher than the natural resolution of the corresponding portions of the eye).

In some embodiments of the method according to the teachings herein, at least some, and in some embodiments all, of the first base image that corresponds to the foveal visual field is preserved in the first display image, that is to say, that for creating of the first display image at least a portion of the first base image that corresponds to a part of the foveal visual field remains unchanged and is not distorted or compressed: pixels from outside the portion of the first base image that corresponds to that part of foveal visual field are not translated to the preserved portion of the first display image that corresponds to that preserved part of the foveal visual field.

Accordingly, in some embodiments, during the creating of the first display image, pixels from portions of the first base image corresponding to at least part of the foveal visual field of the first eye are not translated so that the portions of the first display image corresponding to that part of the foveal visual field of the first eye are the same as the corresponding portion of the first base image. In some such embodiments, the method further comprises: determining the gaze direction of the first eye; and creating and/or displaying the first display image also in accordance with the determined gaze direction so that the portion of the first display image corresponding to that part (the preserved part) of the foveal visual field of the first eye is positioned on the first display screen to be perceived by the foveal visual field of the first eye.

In some embodiments, the portion of the first base image that is preserved in the first display image is the portion that corresponds to the entire foveal visual field of the first eye. In such embodiments, the foveal visual field of the first eye sees the same whether looking at the first display image when displayed or at the first base image (if it were to be displayed). In such embodiments, the only differences perceived by the first eye are portions of the first display image that are located outside of the foveal visual field. Some such embodiments can be considered as compressing portions of a base image that are outside the visual field of the human into non-foveal portions of the display image, thereby providing greater peripheral vision without changing the foveal vision. Such embodiments are useful for increasing the peripheral vision of a human having no visual field deficiency or for a human having tunnel vision that does not affect the foveal visual field.

In some embodiments, the portion of the first base image that is preserved in the first display image is the portion that corresponds to a fraction of the foveal visual field of the first eye, e.g., the preserved portion is not less than 90% of the foveal visual field, not less than 80%, not less than 70%, not less than 50% and even not less than 50% of the foveal visual field.

In some such embodiments, the preserved portion of the images corresponds to the central portion of the foveal visual field and the preserved central portion of the foveal visual field sees the same whether looking at the first display image when displayed or at the first base image (if it was to be displayed). In contrast, the portion of the first display image that corresponds to the non-preserved outer portion of the foveal visual field is different from the portion of the first base image that corresponds to the respective outer portion of the foveal visual field. Some such embodiments can be considered as compressing portions of a base image that are outside the foveal visual field of the human into the outer (peripheral) foveal portions of the display image, thereby sacrificing the outer portion of the foveal visual field but preserving the central portion thereof. Such embodiments are useful for a human having tunnel vision, for instance, by converting some of the foveal visual field to act as a surrogate macular visual field.

It is important to note that in some embodiments of the method according to the teachings herein, a portion of the first base image that corresponds to the foveal visual field is changed and not preserved, for example, pixels from the outside the portion of the first base image that corresponds to the foveal visual field are translated to the portion of the first display image that corresponds to the foveal visual field, or pixels from the portion of the first base image that corresponds to the foveal visual field are translated to a different part of the first display image that corresponds to the foveal visual field. Some such embodiments may include instances where the teachings herein are implemented to display a first display image to be perceived by a human suffering from blind spots in the foveal visual field.

### Macular visual field

The macular visual field is a small (~18° diameter) oval centered in binocular visual field of an eye that surrounds the foveal visual field that provides the central vision called macular vision that is important for visual perception of a human's environment.

Analogously to the described above for the foveal visual field, in some embodiments of the method according to the teachings herein, at least some, and in some embodiments all, of the first base image that corresponds to the macular visual field is preserved in the first display image, that is to say, that for creating of the first display image at least a portion of the first base image that corresponds to a part of the macular visual field remains unchanged and is not distorted or compressed: pixels from outside the portion of the first base image that corresponds to the preserved portion of the macular visual field are not translated to that preserved part of the first display image that corresponds to that preserved part of the macular visual field.

Accordingly, in some embodiments, during the creating of the first display image, pixels from portions of the first base image corresponding to at least part of the macular visual field of the first eye are not translated so the portions of the first display image corresponding to that part of the macular visual field of the first eye are the same as the corresponding portion of the first base image. In some such embodiments, the method further comprises: determining the gaze direction of the first eye; and creating and/or displaying the first display image also in accordance with the determined gaze direction so that the portion of the first display image corresponding to that part of the macular visual field of the first eye is positioned on the first display screen to be perceived by the macular visual field of the first eye.

In some embodiments, the portion of the first base image that is preserved in the first display image is the portion that corresponds to the entire macular visual field of the first eye. In such embodiments, the macular visual field of the first eye sees the same whether looking at the first display image when displayed or at the first base image (if it were to be displayed). The only differences perceived by the first eye are portions of the image that are located outside of the macular visual field. Some such embodiments can be considered as compressing portions of a base image that are outside the visual field of the human into non-macular portions of the display image, thereby providing greater peripheral vision without changing the macular vision. Such embodiments are useful for increasing the peripheral vision of a human having no visual field deficiency.

In some embodiments, the portion of the first base image that is preserved in the first display image is the portion that corresponds to a fraction of the macular visual field of the first eye, e.g., the preserved portion is not less than 80% of the macular visual field, not less than 60%, not less than 50%, not less than 40% and even not less than 30% of the macular visual field.

In some such embodiments, the preserved portion of the images corresponds to the central portion of the macular visual field that surrounds the foveal visual field and the preserved central portion of the macular visual field sees the same whether looking at the first display image when displayed or at the first base image (if it were to be displayed). In contrast, the portion of the first display image that corresponds to the non-preserved outer (peripheral) portion of the macular visual field is different from the portion of the first base image that corresponds to the respective outer portion of the macular visual field. Some such embodiments can be considered as compressing portions of a base image that are outside the macular visual field of the human into the portions of the display image viewed by the outer portion of the macular visual field, thereby sacrificing the outer portion of the macular visual field but preserving the central portion thereof. Such embodiments are useful for a human having tunnel vision, for instance, by converting some of the macular visual field to act as a surrogate peripheral visual field.

It is important to note that in some embodiments of the method according to the teachings herein, a portion of the first base image that corresponds to the macular visual field is changed and not preserved, for example, pixels from the outside the portion of the first base image that corresponds to the macular visual field are translated to the portion of the first display image that corresponds to the macular visual field, or pixels from the portion of the first base image that corresponds to the macular visual field are translated to a different part of the first display image that corresponds to the macular visual field. Some such embodiments may include instances where the teachings herein are implemented to display a first display image to be perceived by a human suffering from a blind spot in the macular visual field. e.g., a macular hole.

### Blind Spots

It is known that some humans have blind spots (e.g., a human suffering from scotomata) so that there are one or more specific locations in the visual field of an eye where eye perceives nothing or perceives a substantially degraded image. Any portion of the visual field is known to be affected by such blind spots, but blind spots are a substantial problem only when the foveal visual field and/or the macular visual field affected.

Some embodiments according to the teachings herein can be useful in mitigating at least some of the inconvenience of such blind spots. Typically, first, the visual field of the first eye of a human suffering from a blind spot is mapped to identify the location of the blind spot. During creation of the first display image to be displayed to that human, pixels from the portions of the first base image that correspond to the blind spot are translated (e.g., radially outwards) to a portion of the first display image that corresponds to a functional portion of the visual field so that substantially no pixels representing the scene are present in portions of the first display image that correspond to the blind spot. The first display image is subsequently displayed on the first display screen with reference to the gaze direction of the eye (as determined above) so that the portion of the first display image that corresponds to the blind spot (therefore including no pixels that represent the scene as a result of the translation) is positioned to be located in the visual field before the blind spot. Since the pixels from the portion of the first base image that corresponds to the blind spot are located in a portion of the first display image that corresponds to functional portions of the visual field of the first eye, the first eye visually perceives the information that would otherwise not be perceived due to the blind spot, albeit in a distorted fashion.

Accordingly, in some embodiments of the method according to the teachings, the visual field of the first eye includes at least one blind spot and at least some of the translation of the pixels is such that substantially no pixels representing the scene are located at a portion of the first display image that corresponds to at least one of the at least one blind spot, and the method further comprises:
determining the gaze direction of the first eye; and
displaying and/or creating the first display image also in accordance with the determined gaze direction so that substantially no pixels representing the scene are located at a portion of the first display image that corresponds to at least one of the at least one blind spots in the visual field of the eye.

In some embodiments, the translation of pixels during the creating of the first display image comprises outward radial translation of pixels away from a point located at a portion of the first display image that corresponds to a point inside the at least one blind spot, in some embodiments a central point. In some embodiments, the outwards radial translating during the creating of the first display image of the pixels away from the point is inhomogeneous, so that the closer a pixel is to the point, the greater the magnitude of the radial translation. In some such embodiments, for any two pixels found on the same radial line radiating from the point, the one of the two pixels closer to the point is radially translated to a greater extent than the further of the two pixels. In some such embodiments, the magnitude of radial translation of a pixel is a continuous function of the distance of the pixel from the point so that the translation is smooth.

### Field of View Compression

As noted above, some embodiments of the teachings herein increase the angular dimensions of the visual field of a human. Specifically, there is a scene which is desired to be viewed by the human that has angular dimensions (horizontal, vertical or both horizontal and vertical) that are larger than the angular dimension of the visual field of the eye so that it is impossible for the human to visually perceive the entire scene at one moment. Such embodiments are useful, for example, for increasing the field of view, especially the peripheral field of view, of a human without a visual field deficiency and also of a human with a limited visual field deficiency, for example, tunnel vision.

Some embodiments of the teachings include starting with a first base image that has relatively large field of view sufficient to represent the entire scene with the large angular dimensions and "compressing" the first base image by translation of pixels (vertically, horizontally, or both vertically and horizontally (e.g., radially) from the periphery of the first base image to create a first display image that represents the scene and then displaying the first display image to the first eye so that the entire field of view of the first display image is perceived by the visual field of the first eye at one time. In some embodiments, some or all of the pixels of the pixels that are translated. are translated horizontally. In some embodiments, some or all of the pixels of the pixels that are translated, are translated vertically. In some embodiments, some or all of the pixels of the pixels that are translated, are translated both vertically and horizontally. In some embodiments, some or all of the pixels of the pixels that are translated both vertically and horizontally are translated radially. In some embodiments, some or all of the pixels of the pixels that are translated both vertically and horizontally are not translated radially.

Accordingly, in some embodiments the base field of view of the first base image has at least one angular dimension greater than the visual field of the first eye; and the translation of the pixels for creation of the first display image is such that the greater angular dimension of the scene is compressed into the display field of view of the first display image.

### Vertical translation

In some such embodiments, the translating of the pixels comprises vertically translating pixels of the first base image towards a horizontal line (e.g., a horizontal center line) in the first base image, thereby compressing the vertical angular dimension of the scene into the display field of view of the first display image. In some such embodiments, the vertical translating of the pixels towards the horizontal line is inhomogeneous so that the further a pixel is from the horizontal line, the greater the magnitude of the vertical translation. In some such embodiments, the magnitude of vertical translation of a pixel is a smooth function of a distance of the pixel from the horizontal line so that the vertical translation of pixels along a vertical line in the base image is devoid of any discontinuity.

### Horizontal translation

In some such embodiments, the translating of the pixels comprises horizontally translating pixels of the first base image towards a vertical line (e.g., a vertical center line) in the first base image, thereby compressing the horizontal angular dimension of the scene into the display field of view of the first display image. In some such embodiments, the horizontal translating of the pixels towards the vertical line is inhomogeneous so that the further a pixel is from the vertical line, the greater the magnitude of the horizontal translation. In some such embodiments, the magnitude of horizontal translation of a pixel is a smooth function of a distance of the pixel from the vertical line so that the horizontal translation of pixels along a horizontal line in the base image is devoid of any discontinuity.

### Radial translation

In some such embodiments, the translating of the pixels comprises radially translating pixels of the first base image towards a point (e.g., a central point) in the first base image, thereby compressing both the horizontal and the vertical angular dimension of the scene into the display field of view of the first display image. In some such embodiments, the radial translating of the pixels towards the point is inhomogeneous so that the further a pixel is from the point, the greater the magnitude of the radial translation. In some such embodiments, the magnitude of radial translation of a pixel is a smooth function of a distance of the pixel from the point so that the radial translation of pixels along a radial line in the base image is devoid of any discontinuity.

### Monocular application of the method

In some embodiments, the method according to the teachings herein is implemented on a single eye only. In some such embodiments, the second eye of the human is absent or blind or completely blind. In some such embodiments, the natural function of the second eye is sufficient, e.g., has no visual field deficiency or no substantial visual field deficiency. In some embodiments, no display screen is positioned before a second eye of the human. In some such embodiments, the second eye is free to function normally without electronic enhancement, optionally with only optical enhancement, e.g., using a lens such as of eyeglasses or a contact lens.

### Binocular application of the method

In some embodiments, the method according to the teachings herein is concurrently implemented on both eyes so that there is a first display screen positioned before the first eye and a second display screen position before the second eye. For brevity, not all of the features and details recited above for the first eye will be repeated hereinbelow for the second eye, but it is explicitly understood that all features and details recited above for the first eye apply to the second eye, mutatis mutandis.

Accordingly, in some embodiments the method further comprises:
a2. positioning a second display screen so that a display surface of the second display screen fills substantially the entire visual field of a second eye of a human and is not visible to the first eye of the human;
b2. at a second display refresh-rate:
   i. from a second video stream extracting a pixelated still second base image having a second base field of view, the second base image representing a second scene, the second base image being digital image data stored in a digital memory,
   ii. from the second base image, creating a pixelated second display image being digital image data (preferably stored in a digital memory) representing the entire second scene, the second display image having a second display field of view different from the second base field of view so that the second scene as represented by the second display image has a field of view not greater than the visual field of the second eye, the creating the second display image comprising while retaining the position of each pixel relative to neighboring pixels translating pixels, each translated pixel from a base coordinate in the second base image to a display coordinate in the second display image; and
   iii. on the second display screen displaying the second display image to the second eye so that substantially the entire field of view of the second display image is perceived by the entire visual field of the second eye,
   thereby allowing the second eye to perceive an entirety of the second scene at one time. In some embodiments, the second display screen is positioned such that relative to the second eye gazing straight ahead, the second display screen fills the visual field of the second eye horizontally not less than 50° nasally, not less than 40° temporally, vertically not less than 40° up and vertically not less than 40° down. In some embodiments, the second display screen fills the visual field of the second eye with angular values limited to the angular values as listed hereinabove for the first display screen.

The positioning of the second display screen while positioning the first display screen is implementable by a person having ordinary skill in the art. As described above, one simple method is by the human wearing a commercially-available VR headset.

In some preferred embodiments, the first display refresh-rate of the first display screen is identical to the second display refresh-rate of the second display screen. That said, in some embodiments, the first display refresh-rate of the first display screen is different from the second display refresh-rate of the second display screen.

### Binocular video streams

In some embodiments, the first and second video streams are the same. In some such embodiments, the video stream is a binocular video stream and each one of the first base image and the second base image are extracted from the appropriate portion of the binocular video stream.

In some embodiments, the first video stream and the second video streams are different. In some embodiments, the first video stream and the second video stream are different and together constitute a binocular pair of video streams, for example, the first video stream is produced by a video camera positioned before the first eye and the second video stream is produced by a video camera positioned before the second eye. In some such embodiments, such a binocular pair is a stereoscopic pair of images.

### Same and different scenes

In some embodiments, the first scene and the second scene are the same, and the first base image and the second base image are the same. In some embodiments, the first scene and the second scene are the same, and the first base image and the second base image are different, being a binocular pair of images of the scene. In some such embodiments, such a binocular pair of base images is a stereoscopic pair of base images.

In some embodiments, the first scene and the second scene are different and the first base image and the second base image are consequently different. For example, in some embodiments, both the first scene and the second scene are of the same region of interest (e.g., a house) but the first scene as represented by the first base image has a wide field of view (e.g., of the entire house) while the second scene as represented by the second base image has a narrower field of view (e.g., of the door of the house).

### Binocular vision

In some embodiments, where the first base image and the second base image are a binocular pair of base images, the first display image and the second display image are a binocular pair of images. In some such embodiments, such a binocular pair of display images is a stereoscopic pair of display images.

### Foveal binocular vision

In some embodiments, where the first scene and the second scene are the same or different (and typically, where at least a part of the first base image and the second base image are a binocular pair of images) the portion of the first display image that corresponds to a part of the foveal visual field of the first eye and the portion of the second display image that corresponds to the respective part of the foveal visual field of the second eye are a binocular pair (in some embodiments, a stereoscopic pair). In some such embodiments, the part of the foveal visual field of the two eyes is not less than 90% of the foveal visual field, not less than 80%, not less than 70%, not less than 50% and even not less than 50% of the foveal visual field. In some such embodiments, other portions of the first and second display image are not binocular pairs and/or not a stereoscopic pair.

In some embodiments, where the first scene and the second scene are the same or different (and typically, where at least a part of the first base image and the second base image are a binocular pair of images), the portion of the first display image that corresponds to the entire foveal visual field of the first eye and the portion of the second display image that corresponds to the entire foveal visual field of the second eye are a binocular pair (and in some embodiments, a stereocopic pair). In some such embodiments, other portions of the first display image and second display image are not binocular pairs and/or not a stereoscopic pair.

### Macular binocular vision

In some embodiments, where the first scene and the second scene are the same or different (and typically, where at least a part of the first base image and the second base image are a binocular pair of images), the portion of the first display image that corresponds to the entire foveal visual field and a part of the macular visual field of the first eye and the portion of the second display image that corresponds to the entire foveal visual field and respective part of the macular visual field of the second eye are a binocular pair, and in some embodiments a stereoscopic pair. In some such embodiments, the part of the macular visual field of the two eyes is not less than 80% of the macular visual field, not less than 60%, not less than 50%, not less than 40% and even not less than 30% of the macular visual field. In some such embodiments, other portions of the first display image and second display image are not binocular pairs and/or not a stereoscopic pair.

In some embodiments, where the first scene and the second scene are the same or different (and typically, where at least a part of the first base image and the second base image are a binocular pair of images), the portion of the first display image that corresponds to the entire foveal visual field and the entire macular visual field of the first eye and the portion of the second display image that corresponds to the entire foveal visual field and the entire macular visual field of the second eye are a binocular pair, and in some embodiments a stereoscopic pair. In some such embodiments, other portions of the first and second display image are not binocular pairs and/or not a stereoscopic pair.

In some embodiments, the differences between the second display image and the second base image are substantially the same as the differences between the first display image and the first base image. For example, such embodiments typically include where both eyes of a human have similar or identical visual field deficiencies.

In some embodiments, the differences between the second display image and the second base image are substantially other than the differences between the first display image and the first base image. For example, such embodiments typically include where one or both eyes includes blind spots.

The second display screen is any suitable display screen, as described hereinabove for the first displayscreen. In some embodiments, the second display screen is positioned so that the second eye directly views the display surface of the second screen, In some embodiments, the second display screen is positioned so that the second eye directly views the display surface of the second screen through an intervening optical component. In some embodiments, the display screen is a virtual retinal display.

In some embodiments, the second display screen is positioned so that the display surface literally fills the entire visual field of the second eye, including when the eye moves relative to the head, up, down, nasally or temporally.

In some embodiments, the second display screen is positioned such that the display surface fills the entire visual field of the second eye when the second eye gazes straight ahead.

In some embodiments, the second display screen is positioned such that the display surface fills the entire mid-peripheral visual field of the second eye when the second eye gazes straight ahead. In some such embodiments, the term "...fills substantially the entire visual field of a second eye of a human second eye..." as used herein means that the second display screen is positioned such that relative to the second eye gazing straight ahead, the second display screen fills the visual field of the second eye horizontally not less than 60° nasally, not less than 60° temporally, vertically not less than 70° up and vertically not less than 80° down. In some such embodiments, the term "...fills substantially the entire visual field of a second eye of a human second eye..." as used herein means that the second display screen is positioned such that relative to the second eye gazing straight ahead, the second display screen fills the visual field of the second eye horizontally not less than 50° nasally, not less than 40° temporally, vertically not less than 40° up and vertically not less than 40° down. In some such embodiments, the term "...fills substantially the entire visual field of a second eye of a human second eye..." as used herein means that the second display screen is positioned such that relative to the second eye gazing straight ahead, the second display screen fills the visual field of the second eye horizontally not less than 60° nasally, not less than 50° temporally, vertically not less than 50° up and vertically not less than 50° down.

Creating the second display image comprises translating pixels each from a base coordinate in the second base image (where the pixel is found in the second base image) to a display coordinate in the display image (where the same pixel is found in the second display image) while retaining the position of each pixel relative to neighboring pixels. The second display image can thus be considered to be a distorted version of the second base image.

The second base image has a base field of view that is, for some reason, not acceptable for display to the second eye as-is (e.g., due to a visual field deficiency of the second eye), typically because the second eye is unable to perceive the entirety of the scene as represented by the second base image (if the second base image was displayed) at one time. The distortion performed is such that the second display image has a display field of view that is different from the base field of view of the second display image, the difference between the display field of view and the base field of view is such that when the second display image is displayed on the second display screen, the entire field of view of the second display image is perceived by the visual field of the second eye at one time, thereby allowing the second eye to perceive the entirety of the scene at one time.

It is important to note that in some embodiments when the method is implemented to display an image for a human having a deficient visual field, the visual field of the human is first mapped and the map is used as a guide for creating the first and second display image, specifically, as which pixels in the respective base image to translate to where in the respective display image.

In preferred embodiments, during creation of a second display image from a second base image, all of the translations of pixels are smooth, that is to say, there are no discontinuities in the second display image. More specifically, a function describing the translation of a series of pixels (e.g., pixels along a line of pixels) is a smooth function that has no discontinuous derivatives.

In some embodiments, the method further comprises: determining the gaze direction of the second eye; and creating and/or displaying the second display image also based one the determined gaze direction.

In some embodiments, not all portions of a second base image are distorted to create the second display image so that these preserved portions are the same in the second base image and the second display image. Accordingly, in some embodiments, during the creating of the second display image, pixels from a specific portion of the second base image are preserved and not translated so that the respective preserved portion of the second display image corresponding to the preserved portions of the second base image are the same.

In some preferred embodiments, the method further comprises: determining the gaze direction of the second eye; and creating and/or displaying the second display image also based one the determined gaze direction so that the preserved portions of the second base image that correspond to specific portions of the visual field of the second eye are perceived by the specific portions of the visual field of the second eye in the second display image.

In some embodiments, during the creating of the second display image, pixels from portions of the second base image corresponding to at least part of the foveal visual field of the second eye are not translated so the portions of the second display image corresponding to that part of the foveal visual field of the second eye are the same as the corresponding portion of the second base image. In some such embodiments, the method further comprises: determining the gaze direction of the second eye; and creating and/or displaying the second display image also in accordance with the determined gaze direction so that the portion of the second display image corresponding to that part of the foveal visual field of the second eye is positioned on the second display screen to be perceived by the foveal visual field of the second eye.

In some embodiments, the portion of the second base image that is preserved in the second display image is the portion that corresponds to the entire foveal visual field of the second eye. In such embodiments, the foveal visual field of the second eye sees the same whether looking at the second display image when displayed or at the second base image (if it were to be displayed). In such embodiments, the only differences perceived by the second eye are portions of the second display image that are located outside of the foveal visual field.

In some embodiments, the portion of the second base image that is preserved in the second display image is the portion that corresponds to a fraction of the foveal visual field of the second eye, e.g., the preserved portion is not less than 90% of the foveal visual field, not less than 80%, not less than 70%, not less than 50% and even not less than 50% of the foveal visual field.

In some such embodiments, the preserved portion of the second images corresponds to the central portion of the foveal visual field of the second eye and the preserved central portion of the foveal visual field sees the same whether looking at the second display image when displayed or at the second base image (if it were to be displayed). In contrast, the portion of the second display image that corresponds to the non-preserved outer portion of the foveal visual field is different from the portion of the second base image that corresponds to the respective outer portion of the foveal visual field. Some such embodiments can be considered as compressing portions of a second base image that are outside the foveal visual field of the human into the outer foveal portions of the second display image, thereby sacrificing the outer portion of the foveal visual field of the second eye but preserving the central portion thereof. Such embodiments are useful for a human having tunnel vision, for instance, by converting some of the foveal visual field to act as a surrogate macular visual field.

It is important to note that in some embodiments of the method according to the teachings herein, a portion of the second base image that corresponds to the foveal visual field is changed and not preserved, for example, pixels from the outside the portion of the second base image that corresponds to the foveal visual field are translated to the portion of the second display image that corresponds to the foveal visual field, or pixels from the portion of the second base image that corresponds to the foveal visual field are translated to a different part of the second display image that corresponds to the foveal visual field. Some such embodiments may include instances where the teachings herein are implemented to display a second display image to be perceived by a human suffering from blind spots in the foveal visual field.

Analogously to the described above for the foveal visual field, in some embodiments of the method according to the teachings herein, at least some, and in some embodiments all, of the second base image that corresponds to the macular visual field of the second eye is preserved in the second display image, that is to say, that for creating of the second display image at least a portion of the second base image that corresponds to a part of the macular visual field of the second eye remains unchanged and is not distorted or compressed: pixels from outside the portion of the second base image that correspond to the preserved portion of the macular visual field of the second eye are not translated to that preserved part of the second display image that corresponds to that preserved part of the macular visual field.

Accordingly, in some embodiments, during the creating of the second display image, pixels from portions of the second base image corresponding to at least part of the macular visual field of the second eye are not translated so the portions of the second display image corresponding to that part of the macular visual field of the second eye are the same as the corresponding portion of the second base image. In some such embodiments, the method further comprises: determining the gaze direction of the second eye; and creating and/or displaying the second display image also in accordance with the determined gaze direction so that the portion of the second display image corresponding to that part of the macular visual field of the second eye is positioned on the second display screen to be perceived by the macular visual field of the second eye.

In some embodiments, the portion of the second base image that is preserved in the second display image is the portion that corresponds to the entire macular visual field of the second eye. In such embodiments, the macular visual field of the second eye sees the same whether looking at the second display image when displayed or at the second base image (if it were to be displayed). The only differences perceived by the second eye are portions of the image that are located outside of the macular visual field. Some such embodiments can be considered as compressing portions of a second base image that are outside the visual field of the second eye of the human into non-macular portions of the second display image, thereby providing greater peripheral vision without changing the macular vision. Such embodiments are useful for increasing the peripheral vision of a human having no visual field deficiency.

In some embodiments, the portion of the second base image that is preserved in the second display image is the portion that corresponds to a fraction of the macular visual field of the second eye, e.g., the preserved portion is not less than 80% of the macular visual field, not less than 60%, not less than 50%, not less than 40% and even not less than 30% of the macular visual field.

In some such embodiments, the preserved portion of the images corresponds to the central portion of the macular visual field of the seconf eye that surrounds the foveal visual field and the preserved central portion of the macular visual field sees the same whether looking at the second display image when displayed or at the second base image (if it wereto be displayed). In contrast, the portion of the second display image that corresponds to the non-preserved outer portion of the macular visual field is different from the portion of the second base image that corresponds to the respective outer portion of the foveal visual field of the second eye. Some such embodiments can be considered as compressing portions of a second base image that are outside the macular visual field of the human into the outer macular portions of the second display image, thereby sacrificing the outer portion of the macular visual field of the second eye but preserving the central portion thereof. Such embodiments are useful for a human having tunnel vision, for instance, by converting some of the macular visual field to act as a surrogate peripheral visual field.

It is important to note that in some embodiments of the method according to the teachings herein, a portion of the second base image that corresponds to the macular visual field of the second eye is changed and not preserved, for example, pixels from the outside the portion of the second base image that corresponds to the macular visual field are translated to the portion of the second display image that corresponds to the macular visual field, or pixels from the portion of the second base image that corresponds to the macular visual field are translated to a different part of the second display image that corresponds to the macular visual field. Some such embodiments may include instances where the teachings herein are implemented to display a second display image to be perceived by a human suffering from a blind spot in the macular visual field of the second eye.

Typically, first the visual field of the second eye of a human suffering from a blind spot is mapped to identify the location of the blind spot. Susbequently, during creation of the second display image to be displayed to that human, pixels from the portions of the second base image that correspond to the blind spot are translated (e.g., radially outwards) . The second display image is subsequently displayed on the second display screen with reference to the gaze direction of the second eye so that the portion of the second display image that corresponds to the blind spot (therefore including no pixels that represent the scene as a result of the translation) is positioned to be located in the visual field before the blind spot. Since the pixels from the portion of the second base image that corresponds to the blind spot are located in a portion of the second display image that corresponds to functional portions of the visual field of the second eye, the second eye visually perceives the information that would otherwise not be perceived due to the blind spot, albeit in a distorted fashion.

Accordingly, in some embodiments of the method according to the teachings herein, the visual field of the second eye includes at least one blind spot and at least some of the translation of the pixels for the creating of the second display image is such that substantially no pixels representing the scene are located at a portion of the second display image that corresponds to at least one of the at least one blind spot, and the method further comprises:
determining the gaze direction of the second eye; and
displaying and/or creating the second display image also in accordance with the determined gaze direction of the second eye so that substantially no pixels representing the scene are located at a portion of the second display image that corresponds to at least one of the at least one blind spots in the visual field of the second eye.

In some embodiments, the translation of pixels for the creating of the second display image comprises outward radial translation of pixels away from a point located at a portion of the second display image that corresponds to a point inside the at least one blind spot, in some embodiments a central point. In some embodiments, the outwards radial translating of the pixels away from the point is inhomogeneous, so that the closer a pixel is to the point, the greater the magnitude of the radial translation. In some such embodiments, for any two pixels found on the same radial line radiating from the point, the one of the two pixels closer to the point is radially translated to a greater extent than the further of the two pixels. In some such embodiments, the magnitude of radial translation of a pixel is a continuous function of the distance of the pixel from the point so that the translation is smooth.

As noted above, some embodiments of the teachings herein increase the angular dimensions of the visual field of a human.In some embodiments, the base field of view of the second base image has at least one angular dimension greater than the visual field of the second eye; and the translation of the pixels for creation of the second display image is such that the greater angular dimension of the scene is compressed into the display field of view of the second display image.

In some such embodiments, the translating of the pixels comprises vertically translating pixels of the second base image towards a horizontal line (e.g., a horizontal center line) in the second base image, thereby compressing the vertical angular dimension of the scene into the display field of view of the second display image. In some such embodiments, the vertical translating of the pixels towards the horizontal line is inhomogeneous so that the further a pixel is from the horizontal line, the greater the magnitude of the vertical translation. In some such embodiments, the magnitude of vertical translation of a pixel is a smooth function of a distance of the pixel from the horizontal line so that the vertical translation of pixels along a vertical line in the base image is devoid of any discontinuity.

In some such embodiments, the translating of the pixels comprises horizontally translating pixels of the second base image towards a vertical line (e.g., a vertical center line) in the second base image, thereby compressing the horizontal angular dimension of the scene into the display field of view of the second display image. In some such embodiments, the horizontal translating of the pixels towards the vertical line is inhomogeneous so that the further a pixel is from the vertical line, the greater the magnitude of the horizontal translation. In some such embodiments, the magnitude of horizontal translation of a pixel is a smooth function of a distance of the pixel from the vertical line so that the horizontal translation of pixels along a horizontal line in the base image is devoid of any discontinuity.

In some such embodiments, the translating of the pixels comprises radially translating pixels of the second base image towards a point (e.g., a central point) in the second base image, thereby compressing both the horizontal and the vertical angular dimension of the scene into the display field of view of the second display image. In some such embodiments, the radial translating of the pixels towards the point is inhomogeneous so that the further a pixel is from the point, the greater the magnitude of the radial translation. In some such embodiments, the magnitude of radial translation of a pixel is a smooth function of a distance of the pixel from the point so that the radial translation of pixels along a radial line in the base image is devoid of any discontinuity.

### Selected specific embodiments of the method

### Tunnel vision

For a human suffering from tunnel vision, radial translation according to the teachings herein is of particular utility for creating a first and a second display image by compressing a first and second base image depicting a scene with a wide angle of view to a first and second display image that is perceivable all at one with the human's limited visual field, where the first and second base image are a binocular pair (and in some embodiments, a stereoscopic pair) and the first and second display image are a binocular pair (and in some embodiments, a stereoscopic pair).

For instance, it is desired to create the two display images from two base images representing a scene having relatively large angular dimensions such as 100° horizontally and 100° vertically to a human having tunnel vision such as **32** depicted in Figure 3 where the human has only a 20° visual field in both eyes corresponding to intact foveal and macular visual fields and with no peripheral visual fields. The pixels of the base images are all radially translated towards the center point of the respective image to create a corresponding display image, effectively resizing or "compressing" the larger base image into the smaller display image. Importantly, in preferred embodiments the radial translating of the pixels towards the center point is inhomogeneous so that the further a pixel is from the center point, the greater the magnitude of the radial translation. Additionally, the magnitude of radial translation of a pixel is a smooth function of a distance of the pixel from the center point so that the radial translation of pixels along a radial line in the base image is devoid of any discontinuity.

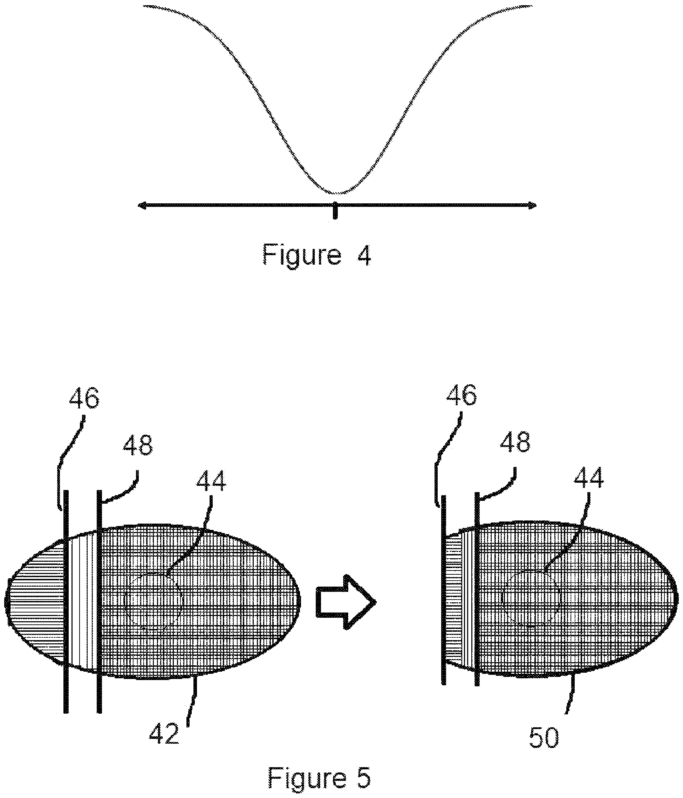

In Figure 4 qualitatively depicts inhomogeneous and smooth translation of pixels in a graph that showing the magnitude of translation of a pixel as a function of the distance of the pixel from the center point of the image. From the graph in Figure 4 it is seen that the translation is inhomogeneous and the further the pixel is from the center point, the more the pixel is translated. From the graph in Figure 4, it is also seen that the magnitude of radial translation of a pixel is a smooth function of a distance of the pixel from the central point so that the radial translation of pixels along a radial line in the base image is devoid of any discontinuity. As a result, the central portion of the display image preserves a relatively high level of detail of the central portion of the base image and is perceived by the portion of the eye that has the highest resolution, while more peripheral portions of the base image are degraded in the display image.

In the embodiment discussed in the immediately preceding paragraphs, a first display image and a second display image constituting a binocular pair of images are created and displayed on a first and second display screen to a human suffering from tunnel vision. In such a way, the human is provided with an artificial visual field including a modicum of binocular peripheral vision, albeit at relatively low resolution, by sacrificing some of the macular visual field. In some embodiments, the human is able to choose to display the first and second images described above, for example when walking around their home or outside or instead choose to switch to display their native tunnel vision devoid of peripheral visual field on the first and second display screens. In some embodiments, the human is able to choose the angular width of the base image, allowing the human to choose between a narrower or broader artificial visual field.

In some embodiments of the teachings herein that are implemented to display an image for a human suffering from tunnel vision, the teachings are implemented monocularly instead of binocularly as discussed in the paragraphs above, that is to say, only a first display image is created and displayed on a first display screen. In such cases, the human loses binocular vision but can simultaneously look at the first screen using a first eye to perceive objects in the relatively broad artificial visual field afforded by the first display image and look at specific objects with the native tunnel vision of the second eye.

### Single eye vision

In some instances horizontal translation of pixels according to the teachings herein is of particular utility to help a human suffering from single-eye vision by creating a first display image by compressing a first base image depicting a scene with a wide field of view into a first display image that is perceivable all at once with the human's limited natural visual field resulting from having only a single functioning eye.

Such an embodiment is discussed with reference to Figure 5. In Figure 5, oval **42** represents a first base image of a scene that corresponds to a complete visual field of a two-eyed human without visual field deficiency, circle **44** indicates the portion of the first base image that corresponds to the macular visual field and vertical line **46** indicates the left limit of the visual field of the one-eyed human having only a functioning right eye who is unable to visually perceive anything to the left of vertical line **46** due to the presence of the nose. Truncated oval **50** represents a first display image created from the first base image where the field of view of the first display image is the same as the visual field of the human suffering from single-eye vision.

In accordance with an embodiment of the teachings herein, a vertical line **48** is defined which is to the right of the portion of the base image that corresponds to the left visual field limit **46** and does not pass through the portion of the first base image that corresponds to the macular visual field **44.** To create the first display image, all the pixels of the base image that are to the left of vertical line **48** are horizontally translated rightwards to a degree that all the pixels are located to the right of the left visual field limit **46.**

In the resulting display image, the macular and foveal visual fields are unaffected, as well as most of the peripheral vision. Only pixels in portions of the base image to the left of vertical line **48** are translated, so that most of the resulting display image is undistorted, but the human is provided with an artificial visual field that has angular dimensions similar to those of a normal visual field.

Similarly to the discussed above with reference to tunnel vision, in preferred embodiments the horizontal translating of the pixels towards vertical line **48** is inhomogeneous so that the further a pixel is from vertical line **48**, the greater the magnitude of the horizontal translation. Additionally, the magnitude of horizontal translation of a pixel is a smooth function of a distance of the pixel from horizontal line **48** so that the horizontal translation of pixels along a horizontal line in the base image is devoid of any discontinuity.

A graph that qualitatively depicts the magnitude of translation of a pixel as a function of the distance of the pixel from vertical line **48** would look like the left half of the graph of Figure 4.

### Hemianopsia

For a human suffering from hemianopsia, in some instances a combination of vertical and horizontal translation of pixels according to the teachings herein is of particular utility for creating a first and a second display image.by compressing a binocular pair of a first and a second base image depicting a scene with a normal field of view to a binocular pair of first and second display image that is perceivable all at once with the human's limited visual field.

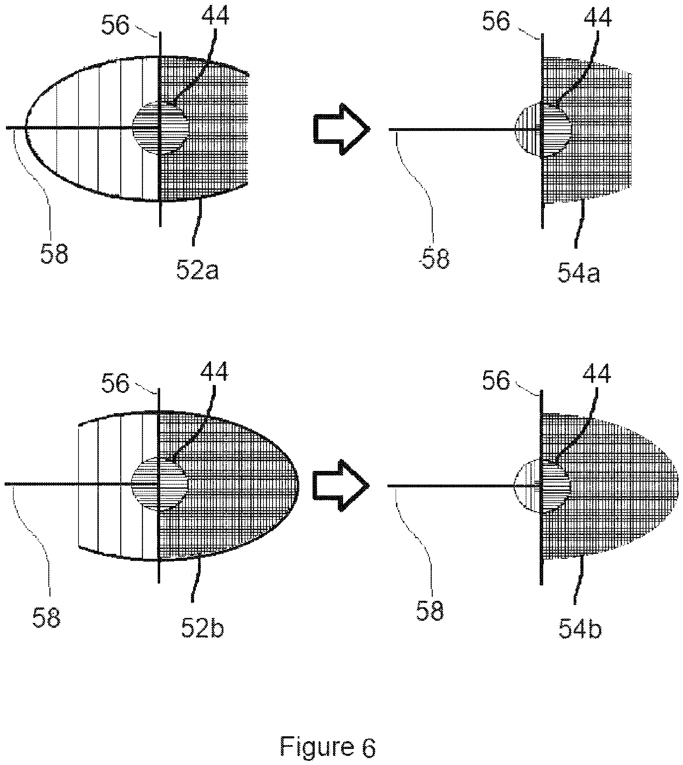

Such an embodiment is discussed with reference to Figure 6.

In Figure 6, truncated oval **52a** represents a first base image of a scene that corresponds to a visual field of a left eye without visual field deficiency while truncated oval **52b** represents a second base image of a scene that corresponds to a visual field of a right eye without visual field deficiency. **52a** and **52b** are a binocular pair of base images.

Truncated oval **54a** represents the first display image created from the first base image **52a** where the field of view of the first display image is the same as the visual field of the left eye of the human suffering from the hemianopsia.

Truncated oval **54b** represents the second display image created from the second base image **52b** where the field of view of the second display image is the same as the visual field of the right eye of the human suffering from the hemianopsia.

In accordance with an embodiment of the teachings herein, a vertical line **56** is defined which passes through the portion of the base images **52a** and **52b** that corresponds to the macular visual fields but just to the left of the portion that corresponds to the foveal visual field. Further, a horizontal line **58** is defined which passes through the horizontal mid-line of base images **52a** and **52b.**

To create the first display image **54a** from first base image **52a**, all the pixels of the base image that are to the left of vertical line **56** and above horizontal line **58** are horizontally transferred rightwards towards vertical line **56** and vertically transferred downwards towards horizontal line **58** into the portion of first display image that corresponds to the macular visual field. Similarly, all the pixels of the base image that are to the left of vertical line **56** and below horizontal line **58** are horizontally transferred rightwards towards vertical line **56** and vertically transferred upwards towards horizontal line **58** into the portion of first display image that corresponds to the macular visual field.

To create the second display image **54b** from the second base image **52b**, pixels of the second base image are horizontally and vertically translated substantially as described above. Preferably the translation of the pixels of the second base image **52b** is done in such a way so that the resulting first and second display images **54a** and **54b** are a binocular pair.

The portions of the resulting binocular pair of display images that correspond to the right portion of the visual field of the human which functions normally is unchanged. In contrast, the portions of the resulting binocular pair of display images that correspond to the left portion of the visual field of the human which is deficient is changed. Specifically, the portions of the two base images that correspond to the left peripheral visual field are compressed by a combination of horizontal and vertical translation into the portions of the respective display images that correspond to the left macular visual field.

Similarly to the discussed above in preferred embodiments the horizontal translating of the pixels towards vertical line **56** is inhomogeneous so that the further a pixel is from vertical line **56**, the greater the magnitude of the horizontal translation. Additionally, the magnitude of horizontal translation of a pixel is a smooth function of a distance of the pixel from horizontal line **56** so that the horizontal translation of pixels along a horizontal line in the base image is devoid of any discontinuity. Analogously, the vertical translating of the pixels towards horizontal line **58** is inhomogeneous so that the further a pixel is from horizontal line **58**, the greater the magnitude of the vertical translation. Additionally, the magnitude of vertical translation of a pixel is a smooth function of a distance of the pixel from vertical line **56** so that the vertical translation of pixels along a vertical line in the base image is devoid of any discontinuity.

### Enhanced visual field

As mentioned hereinabove, some embodiments of the teachings herein are optionally used to enhance the visual field of a human, including a human having no visual field deficiencies.

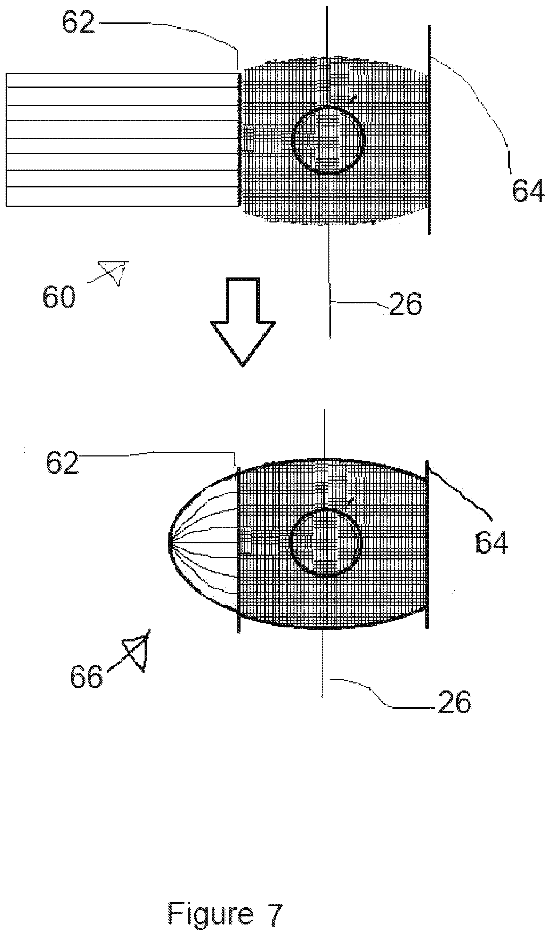

Such an embodiment is discussed with reference to Figure 7 for the left eye of a human.

In Figure 7, 60 represents a first base image of a scene that has a horizontal field of view of 65° nasally and 180° temporally. Vertical line **62** is located 65° temporally relative to vertical meridian **26** and vertical line **64** is located 65° nasally relative to vertical meridian **26** so that if a human having a normal visual field was to look at the scene represented by **60**, the portion of **60** between lines **62** and **64** would correspond to the binocular visual field of the human.

When it is desired to provide the human with a visual field of 360° horizontally, where each eye has a 180° visual field horizontally and a usual 110° horizontally binocular visual field, a first display image **66** is created from first base image **60** where the portions of **60** between lines **62** and **64** are preserved and not changed in first display image **66**, but the pixels of the entire portion of first base image **60** to the left of vertical line **62** are horizontally and vertically translated as described above so as to compress the pixels into the portion of first display image that corresponds to the non-binocular far peripheral visual field of the left eye. A second display image is created in an analogous way from a second base image (both not depicted) for display to the right eye of the human. When the first display image is displayed to the left eye and the second display image is displayed to the left eye of the human, the human has a normal unchanged 110° horizontal binocular visual field, However, instead of the far peripheral visual field that allows perception of about 30° horizontally to either side of the binocular visual field, the human has visual perception of 125° horizontally to either side of the 110° binocular visual field.

### Image display devices according to the teachings herein

The methods according to the teachings herein may be implemented using any suitable device. In some preferred embodiments, the methods are implemented on commercially-available VR headsets, preferably such VR headsets with eye-tracking components and functionality, where the processor of the headset is configured using the required software, firmware and/or hardware to implement the methods according to the teachings herein. A person having ordinary skill in the art of VR is able to make the required modifications to implement the teachings herein upon perusal of the specification.

In some embodiments, it is preferred to use a device specifically made for implementing the teachings herein. A person having ordinary skill in the art of VR is able to design and build such a specifically-made device to implement the teachings herein upon perusal of the specification.

### Monocular headset

In some embodiments, a device according to the teachings herein is a monocular device. Thus, according to an aspect of some the embodiments of the teachings herein, there is provided a monocular headset configured to be worn on the head of a human, comprising:
a single display screen mounted on a headset body, so that when the headset body is worn on the head of a human, the display screen is positioned so that a display surface of the display screen fills substantially the entire visual field of a first eye of a human and is not visible to a second eye of the human (when the second eye is open and functioning); and
functionally associated with the display screen, a digital processor including a video input port configured to accept a video stream via the video input port and to implement any suitable embodiments of the methods according to the teachings herein with the video stream using the display screen,
optionally the headset further comprising an eye-tracker to determine the gaze direction of the first eye and to provide the determined gaze direction to the processor.

In some embodiments, the monocular headset further comprises a digital video camera with a video outlet port functionally associated with the video input port of the processor, the video camera configured to acquire video images and to output a digital video stream corresponding to the video images via the video outlet port to the processor. In some embodiments, the video camera is physically attached to the headset body. In some embodiments, the video camera is physically attached to the headset body before the display screen. Any suitable video camera may be used for implementing such a headset, for example, a video camera as is known in the art of smartphones.

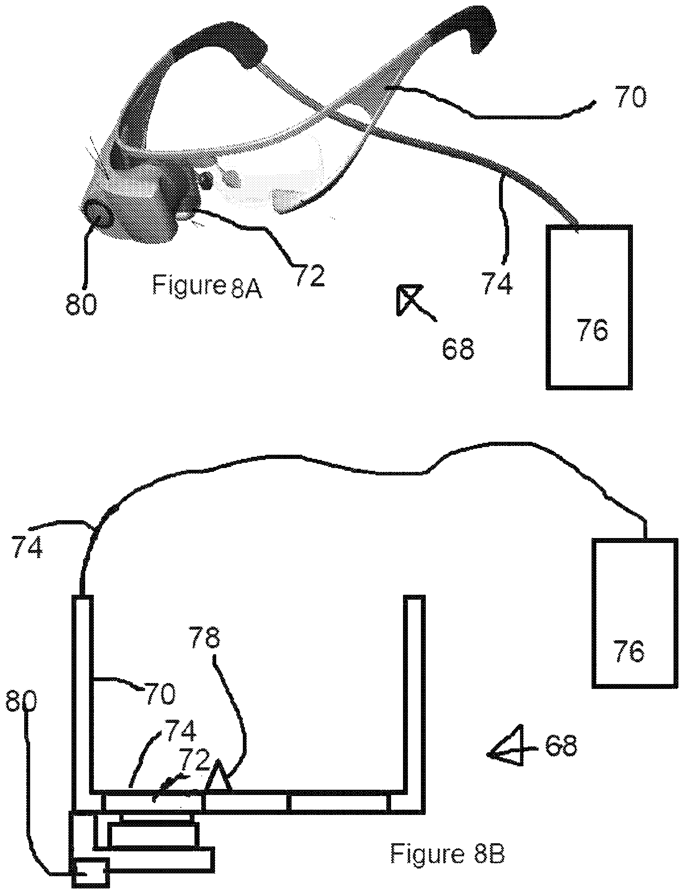

An embodiment of a monocular headset **68** according to an embodiment of the teachings herein is schematically depicted in Figure 8A in perspective view and in Figure 8B in schematic top cross section. Headset **68** includes a headset body **70**, a single display screen **72** mounted on headset body **70** so that when headset body **70** is worn on the head of a human, display screen **72** is positioned so that a display surface **74** of display screen **72** fills substantially the entire visual field of a right eye of the human and is not visible to the left eye of the human. Functionally associated with display screen **72** through cable **74** is digital processor **76** that includes a video input port (not depicted). Digital processor **74** is configured to accept a video stream via the video input port and to implement any suitable method as described herein such as displaying a created display image using display screen **72.** Headset **68** further comprises an eye-tracker **78** to determine the gaze direction of a right eye when the headset is worn and to provide the determined gaze direction to processor **74** through cable **74.** Headset **68** further comprises a digital video camera **80** with a video outlet port (not depicted) that is functionally associated with the video input port of digital processor **76** through cable **74.** Video camera **80** is positioned before display screen **72** and is configured to acquire video images from in front of a wearer and to output a digital video stream corresponding to the acquired video images via the video outlet port through cable **74** to digital processor **76.**

### Binocular headset

In some embodiments, a device according to the teachings herein is a binocular device. Thus, according to an aspect of some the embodiments of the teachings herein, there is provided a binocular headset configured to be worn on the head of a human, comprising:
a first display screen mounted on a headset body, so that when the headset body is worn on the head of a human, the first display screen is positioned so that a display surface of the first display screen fills substantially the entire visual field of a first eye of a human and is not visible to a second eye of the human (when the second eye is open and functioning);
a second display screen mounted on the headset body, so that when the headset body is worn on the head of a human, the second display screen is positioned so that a display surface of the second display screen fills substantially the entire visual field of a second eye of a human and is not visible to the first eye of the human (when the first eye is open and functioning); and
functionally associated with said first display screen and said second display screen, a digital processor including a video input port configured to accept a video stream via the video input port and to implement any suitable embodiment of the methods according to the teachings herein with the video stream using the first display screen and the second display screen,
optionally the headset further comprising an eye-tracker to determine the gaze direction of the first eye and of the second eye, and to provide the determined gaze directions to the processor.

In some embodiments, the binocular headset further comprises: a digital binocular video camera with a video outlet port functionally associated with the video input port of the processor, the binocular video camera configured to acquire binocular pairs of monocular video images and to output a digital binocular video stream corresponding to the video images via the video outlet port to the processor. In some embodiments, the digital binocular video camera is physically attached to the headset body, and is optionally attached so that each one of the monocular video streams of the binocular video images is acquired from before a different one of a first eye and a second eye of a human wearing the headset.

In some embodiments, the binocular headset further comprises:
a first digital video camera with a first video outlet port functionally associated with the video input port of the processor, the first video camera configured to acquire monocular video images and to output a first monocular digital video stream corresponding to the monocular video images via the video outlet port to the processor, and
a second digital video camera with a second video outlet port functionally associated with the video input port of the processor, the second video camera configured to acquire monocular video images and to output a second monocular digital video stream corresponding to the monocular video images via the video outlet port to the processor, In some such embodiments, the first digital video camera and the second digital video camera are physically attached to the headset body, In some embodiments, the first digital video camera is physically attached to the headset body before the first display screen; and the second digital video camera is physically attached to the headset body before the second display screen, so that a video stream output of the first digital video camera and a video stream output of the second digital video camera taken together constitute a binocular pair.

Any suitable video camera may be used for implementing such a binocular headset, for example, a video camera as is known in the art of smartphones.

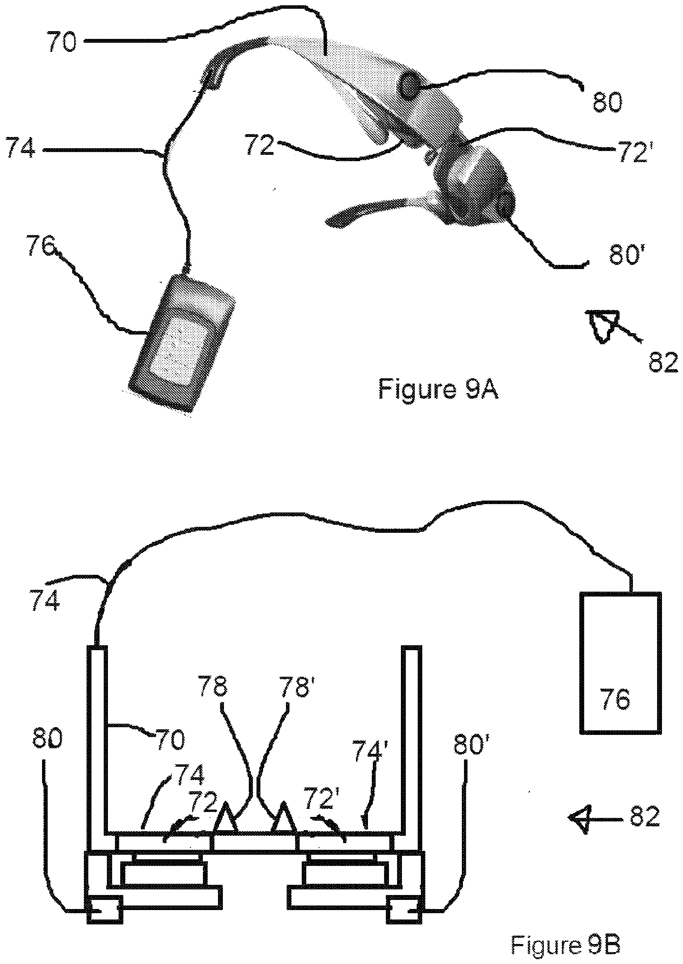

An embodiment of a binocular headset **82** according to an embodiment of the teachings herein is schematically depicted in Figure 9A in perspective view and in Figure 9B in schematic top cross section. Headset **82** is similar to headset **68** discussed with reference to Figures 8 and includes many of the same components. Additionally, headset **82** includes a second display screen **72'** mounted on headset body **70** so that when headset body **70** is worn on the head of a human, second display screen **72'** is positioned so that a display surface **74'** of second display screen **72'** fills substantially the entire visual field of a left eye of the human and is not visible to the right eye of the human. Second display screen **72'** is functionally associated with digital processor **76** through cable **74.** Headset **82** further comprises a second eye-tracker **78'** to determine the gaze direction of a left eye when headset **82** is worn and to provide the determined gaze direction to processor **76.** Headset **82** further comprises a second digital video camera **80'** with a video outlet port (not depicted) that is functionally associated with the video input port of digital processor **76** through cable **74**, Video camera **80'** is positioned before display screen **72'** and is configured to acquire video images from in front of a wearer and to output a digital video stream corresponding to the acquired video images via the video outlet port through cable **74** to digital processor **76.** The relative positions of video cameras **80** and **80'** are such that, all other things being equal, an image acquired by video camera **80** and an image simultaneously acquired by video camera **80'** constitute a binocular pair of images.

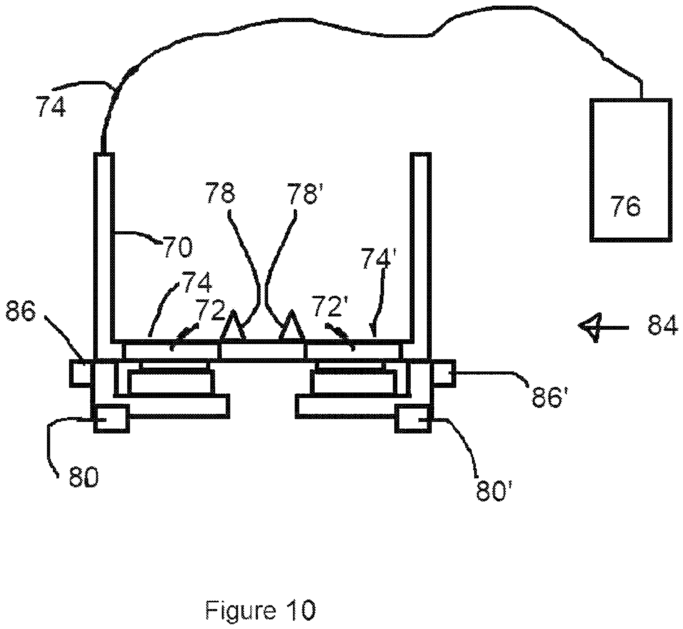

An additional embodiment of a binocular headset according to an embodiment of the teachings herein, headset **84** is schematically depicted in Figure 10 in schematic top cross section. Headset **84** is similar to headset **82** discussed with reference to Figures 8 and includes all of the same components. Additionally, headset **84** includes two additional digital video cameras, right side-camera **86** and left side-camera **86'.** Both side-cameras include a full-frame fisheye lens that has a 147° horizontal field of view and a 94° vertical field of view. The four cameras **80**, **80'**, **86** and **86'** allow 360° horizontal field of view image acquisition (although there is a blind spot directly behind and close to a human wearing headset **84** due to the line of sight obstruction caused by the back of the head of the human).

A headset such as headset **84** is particularly useful for implementing embodiments of the teachings herein for enhancing a human's visual field beyond the normal.

As noted above, a digital processor of a headset according to the teachings herein is functionally associated with at least one display screen. In some embodiments, the functional association is through wireless communication. That said, due to the large amount of data that the digital processor is required to transfer to each display screen, in preferred embodiments the digital processor is functionally associated with at least one display screen through a physical component such as a communication cable, as depicted in Figures 8, 9 and 10.

The power source of a headset according to the teachings herein is any suitable power source. In preferred embodiments, the power source is attached to the headset. In some embodiments, the power source is worn by the human (e.g., in a backpack) and the power source provides power to the various components of the headset, for example, through an electrical cable. In Figures 8, 9 and 10, the power sources of the respective headsets are rechargeable batteries held in the same physical package as digital processor **76.**

In some embodiments, the processor of a headset is configured to create a display image from a respective base image in a single specific user-unchangeable way.

In some embodiments, the processor of a headset is configured to create a display image from a respective base image in more than one specific way, for example, a first way is for when the human watches television or reads and does not require substantial peripheral vision, a second way is for when the human walks around their home and requires some peripheral vision, and a third way is for when the human walks outside their home and needs to be aware of unfamiliar surroundings via more extensive peripheral vision. In some such embodiments, the user can select the way the processor creates a display image from a respective base image. In some embodiments, the selection of the way the processor creates a display image from a respective base image is automatic, for example, based on the distance from the headset to an object before the headset.

In some embodiments, the way or ways the creation of a display image from a respective base image is based on a diagnosis of a specific visual field deficiency of the human who is intending to use the headset. In some such embodiments, a health-care professional such as an ophthalmologist determines the actual visual field of one or both of the eyes of the human, for example, using methods known in the art, and using the determined visual field or fields, provides one or more desired ways to create a display image from a base image.

Unless otherwise defined, all technical and scientific terms used herein have the same meaning as commonly understood by one of ordinary skill in the art to which the invention pertains. In case of conflict, the specification, including definitions, takes precedence.

As used herein, the terms "comprising", "including", "having" and grammatical variants thereof are to be taken as specifying the stated features, integers, steps or components but do not preclude the addition of one or more additional features, integers, steps, components or groups thereof.

As used herein, the indefinite articles "a" and "an" mean "at least one" or "one or more" unless the context clearly dictates otherwise.

As used herein, when a numerical value is preceded by the term "about", the term "about" is intended to indicate +/-10%.

As used herein, a phrase in the form "A and/or B" means a selection from the group consisting of (A), (B) or (A and B). As used herein, a phrase in the form "at least one of A, B and C" means a selection from the group consisting of (A), (B), (C), (A and B), (A and C), (B and C) or (A and B and C).

As used herein and in the priority document, the terms "apparatus" and "device" are synonomous.

As used herein, a binocular pair of images is a pair of images where a portion of each one of the two images can be combined to provide stereopsis. If the entirety of the two images can be combined to provide stereopsis, then the images are a stereoscopic pair of images. The image acquired by the left eye of a human and the image acquired by the right eye of the human together form a binocular pair of images.

In some embodiments of the teachings herein, a video camera is described as being positioned before an eye. As used herein, when a video camera is positioned before a left eye, the camera is positioned left of the sagittal plane of the head and within 10 cm of an imaginary line that passes through the left eye and is perpendicular to the coronal plane of the head, the video camera directed to acquire images from substantially the same direction as the left eye. When a video camera is positioned before a right eye, the camera is positioned right of the sagittal plane of the head and within 10 cm of an imaginary line that passes through the right eye and is perpendicular to the coronal plane of the head, the video camera being directed to acquire images from substantially the same direction as the right eye.

As used herein, for clarity the term "image" refers to a visible image (e.g., as displayed on permanent media such as on printed paper or electronic media such as a display screen (LED, LCD, CRT)), as well as data (especially electronic data) representing the image including data stored, for example, on magnetic or electrical media (e.g., flash memory, magnetic disk, magnetic tape).

As used herein, for clarity the term "pixel" refers to an element making up a pixelated image (displayed or stored as data) and also to the value of the pixel, as the context dictates.

Embodiments of methods and/or devices described herein may involve performing or completing selected tasks manually, automatically, or a combination thereof. Some methods and/or devices described herein are implemented with the use of components that comprise hardware, software, firmware or combinations thereof. In some embodiments, some components are general-purpose components such as general purpose computers or digital processors. In some embodiments, some components are dedicated or custom components such as circuits, integrated circuits or software.

For example, in some embodiments, some of an embodiment is implemented as a plurality of software instructions executed by a data processor, for example which is part of a general-purpose or custom computer. In some embodiments, the data processor or computer comprises volatile memory for storing instructions and/or data and/or a non-volatile storage, for example, a magnetic hard-disk and/or removable media, for storing instructions and/or data. In some embodiments, implementation includes a network connection. In some embodiments, implementation includes a user interface, generally comprising one or more of input devices (e.g., allowing input of commands and/or parameters) and output devices (e.g., allowing reporting parameters of operation and results.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub combination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the invention.

Section headings are used herein to ease understanding of the specification and should not be construed as necessarily limiting.

## Claims

1. An image-display method comprising:
a1. positioning a first display screen so that a display surface of said first display screen fills substantially the entire visual field of a first eye of a human and is not visible to a second eye of said human;
b1. at a first display refresh-rate:
i. from a first video stream extracting a pixelated still first base image having a first base field of view, said first base image representing a first scene, said first base image being digital image data stored in a digital memory;
ii. from said first base image, creating a pixelated first display image being digital image data representing said entire first scene, said first display image having a first display field of view different from said first base field of view, so that said first scene as represented by said first display image has a field of view not greater than the visual field of said first eye, said creating said first display image comprising while retaining the position of each pixel relative to neighboring pixels translating pixels, each translated pixel from a base coordinate in said first base image to a display coordinate in said first display image, and
iii. on said first display screen displaying said first display image to said first eye so that the entire said first field of view of said first display image is perceived by the visual field of said first eye at one time,
thereby allowing said first eye to perceive an entirety of said first scene at one time
wherein the visual field of said first eye includes at least one blind spot and at least some of said translation of said pixels is such that substantially no pixels representing said scene are located at a portion of said first display image that corresponds to at least one of said at least one blind spot, the method further comprising:
determining a gaze direction of said first eye; and
displaying and/or creating said first display image also in accordance with said determined gaze direction so that substantially no pixels representing said scene are located at a portion of said first display image that corresponds to at least one of said at least one blind spots in the visual field of said first eye.

2. The method of claim 1, wherein during said creating of said first display image, pixels from a specific portion of said first base image are preserved and not translated so that the preserved portion of said first display image corresponding to said preserved portion of said first base image are the same.

3. The method of any one of claims 1 to 2, wherein during said creating of said first display image, pixels from portions of said first base image corresponding to at least part of the foveal visual field of said first eye are not translated so that said portions of said first display image corresponding to said part of the foveal visual field of said first eye are the same as the corresponding portion of said first base image.

4. The method of any one of claims 1 to 3, wherein during said creating of said first display image, pixels from portions of said first base image corresponding to the macular visual field of said first eye are not translated so the portions of said first display image corresponding to the macular visual field of said first eye are the same as the corresponding portion of said first base image.

5. The method of any one of claims 1 to 4,
wherein said translation of pixels during said creating of said first display image comprises outward radial translation of pixels away from a point located at a portion of said first display image that corresponds to a point inside said at least one blind spot.

6. The method of any one of claims 1 to 5, wherein said base field of view has at least one angular dimension greater than the visual field of said first eye; and
said translation of pixels for said creating of said first display image is such that said greater angular dimension of said scene is compressed into said display field of view of said first display image;
wherein said translation of pixels comprises vertically translating pixels of said first base image towards a horizontal line in said first base image, thereby compressing a vertical angular dimension of said scene into said display field of view of said first display image, and wherein said vertical translating of pixels towards said horizontal line is inhomogeneous so that the further a pixel is from said horizontal line, the greater the magnitude of said vertical translation.

7. The method of claim 6, wherein the magnitude of vertical translation of a pixel is a smooth function of a distance of said pixel from said horizontal line so that said vertical translation of pixels along a vertical line in said base image is devoid of any discontinuity.

8. The method of any one of claims 1 to 7, wherein said base field of view has at least one angular dimension greater than the visual field of said first eye; and
said translation of pixels for said creating of said first display image is such that said greater angular dimension of said scene is compressed into said display field of view of said first display image;
wherein said translation of pixels comprises horizontally translating pixels of said first base image towards a vertical line in said first base image, thereby compressing a horizontal angular dimension of said scene into said display field of view of said first display image, wherein said horizontal translating of pixels towards said vertical line is inhomogeneous so that the further a pixel is from said vertical line, the greater the magnitude of said horizontal translation.

9. The method of any one of claims 1 to 8, wherein said base field of view has at least one angular dimension greater than the visual field of said first eye; and
said translation of pixels for said creating of said first display image is such that said greater angular dimension of said scene is compressed into said display field of view of said first display image;
wherein said translation of pixels comprises radially translating pixels of said first base image towards a point in said first base image, thereby compressing both a horizontal and a vertical angular dimension of said scene into said display field of view of said first display image, wherein said radial translating of pixels towards said point is inhomogeneous so that the further a pixel is from said point, the greater the magnitude of said radial translation.

10. The method of any one of claims 1 to 9, wherein no display screen is positioned before said second eye of said human.

11. The method of any one of claims 1 to 10, further comprising:
a2. positioning a second display screen so that a display surface of said second display screen fills substantially the entire visual field of a second eye of a human and is not visible to said first eye of said human;
b2. at a second display refresh-rate:
i. from a second video stream extracting a pixelated still second base image having a second base field of view, said second base image representing a second scene, said second base image being digital image data stored in a digital memory,
ii. from said second image, creating a pixelated second display image being digtal image data representing said entire second scene, said second display image having a second display field of view different from said second base field of view, so that said second scene as represented by said second display image has a field of view not greater than the visual field of said second eye, said creating said second display image comprising while retaining the position of each pixel relative to neighboring pixels translating pixels, each translated pixel from a base coordinate in said second base image to a display coordinate in said second display image, and
iii. on said second display screen displaying said second display image to said second eye so that the entire said second field of view of said second display image is perceived by the visual field of said second eye at one time,
thereby allowing said second eye to perceive an entirety of said second scene at one time.

12. The method of claim 11, wherein said first scene and said second scene are different.

13. The method of any one of claims 11 to 12, wherein a portion of said first display image that corresponds to the entire foveal visual field and a part of the macular visual field of said first eye and a portion of said second display image that corresponds to the entire foveal visual field and respective part of the macular visual field of said second eye are a binocular pair, and other portions of said first display image and said second display image are not binocular pairs.

14. A monocular headset configured to be worn on the head of a human, comprising:
a single display screen mounted on a headset body, so that when said headset body is worn on the head of a human, said display screen is positioned so that a display surface of said display screen fills substantially the entire visual field of a first eye of a human and is not visible to a second eye of said human; and
functionally associated with said display screen, a digital processor including a video input port configured to accept a video stream via said video input port and to implement the method of any one of claims 1 to 10 with a said video stream using said display screen, further comprising an eye-tracker to determine the gaze direction of a said first eye and to provide the determined gaze direction to said processor.

15. A binocular headset configured to be worn on the head of a human, comprising:
a first display screen mounted on a headset body, so that when said headset body is worn on the head of a human, said first display screen is positioned so that a display surface of said first display screen fills substantially the entire visual field of a first eye of a human and is not visible to a second eye of said human;
a second display screen mounted on said headset body, so that when said headset body is worn on the head of a human, said second display screen is positioned so that a display surface of said second display screen fills substantially the entire visual field of a second eye of a human and is not visible to a said first eye of said human; and
functionally associated with said first display screen and with said second display screen, a digital processor including a video input port configured to accept a video stream via said video input port and to implement the method of any one of claims 11 to 13 with a said video stream using said first display screen and said second display screen, further comprising an eye-tracker to determine the gaze direction of a said first eye and of a said second eye, and to provide the determined gaze directions to said processor.

## Patentansprüche

1. Bildanzeigeverfahren, umfassend:
a1. Positionieren eines ersten Anzeigebildschirms, sodass eine Anzeigefläche des ersten Anzeigebildschirms im Wesentlichen das gesamte Gesichtsfeld eines ersten Auges eines Menschen ausfüllt und für ein zweites Auge des Menschen nicht sichtbar ist;
b1. mit einer ersten Anzeige-Auffrischungsrate:
i. Extrahieren, aus einem ersten Videostrom, eines gepixelten, unbewegten ersten Basisbilds, das ein erstes Basis-Sichtfeld aufweist, wobei das erste Basisbild eine erste Szene darstellt und das erste Basisbild digitale Bilddaten ist, die in einem digitalen Speicher gespeichert sind;
ii. Erzeugen, aus dem ersten Basisbild, eines gepixelten ersten Anzeigebilds, das digitale Bilddaten ist, die die gesamte erste Szene darstellen, wobei das erste Anzeigebild ein erstes Anzeige-Sichtfeld aufweist, das sich von dem ersten Basis-Sichtfeld unterscheidet, sodass die erste Szene, wie sie durch das erste Anzeigebild dargestellt ist, ein Sichtfeld aufweist, das nicht größer ist als das Gesichtsfeld des ersten Auges, wobei ein Erzeugen des ersten Anzeigebilds unter Beibehaltung der Position von jedem Pixel in Bezug auf benachbarte Pixel ein Verschieben von Pixeln umfasst, jedes verschobene Pixel von einer Basiskoordinate in dem ersten Basisbild zu einer Anzeigekoordinate in dem ersten Anzeigebild, und
iii. Anzeigen, auf dem ersten Anzeigebildschirm, des ersten Anzeigebilds für das erste Auge, sodass das gesamte erste Sichtfeld des ersten Anzeigebilds von dem Gesichtsfeld des ersten Auges zu einem Zeitpunkt wahrgenommen wird,
wodurch dem ersten Auge ermöglicht wird, eine Gesamtheit der ersten Szene zu einem Zeitpunkt wahrzunehmen, wobei das Gesichtsfeld des ersten Auges mindestens einen blinden Fleck beinhaltet und mindestens ein Teil der Verschiebung der Pixel derart ist, dass sich im Wesentlichen keine Pixel, die die Szene darstellen, in einem Abschnitt des ersten Anzeigebilds befinden, der mindestens einem von dem mindestens einen blinden Fleck entspricht, das Verfahren ferner umfassend:
Bestimmen einer Blickrichtung des ersten Auges; und
Anzeigen und/oder Erzeugen des ersten Anzeigebilds auch in Übereinstimmung mit der bestimmten Blickrichtung, sodass sich im Wesentlichen keine Pixel, die die Szene darstellen, in einem Abschnitt des ersten Anzeigebilds befinden, der mindestens einem von dem mindestens einen blinden Flecken in dem Gesichtsfeld des ersten Auges entspricht.

2. Verfahren nach Anspruch 1, wobei beim Erzeugen des ersten Anzeigebilds Pixel aus einem bestimmten Teil des ersten Basisbilds beibehalten bleiben und nicht verschoben werden, sodass der beibehaltene Abschnitt des ersten Anzeigebilds, der dem beibehaltenen Abschnitt des ersten Basisbilds entspricht, derselbe ist.

3. Verfahren nach einem der Ansprüche 1 bis 2, wobei beim Erzeugen des ersten Anzeigebilds Pixel von Abschnitten des ersten Basisbilds, die zumindest einem Teil des fovealen Gesichtsfelds des ersten Auges entsprechen, nicht verschoben werden, sodass die Abschnitte des ersten Anzeigebilds, die dem Teil des fovealen Gesichtsfelds des ersten Auges entsprechen, gleich sind wie der entsprechende Abschnitt des ersten Basisbilds.

4. Verfahren nach einem der Ansprüche 1 bis 3, wobei beim Erzeugen des ersten Anzeigebilds Pixel von Abschnitten des ersten Basisbilds, die dem makularen Gesichtsfeld des ersten Auges entsprechen, nicht verschoben werden, sodass die Abschnitte des ersten Anzeigebilds, die dem makularen Gesichtsfeld des ersten Auges entsprechen, gleich sind wie der entsprechende Abschnitt des ersten Basisbilds.

5. Verfahren nach einem der Ansprüche 1 bis 4,
wobei die Verschiebung von Pixeln beim Erzeugen des ersten Anzeigebilds eine nach außen gerichtete radiale Verschiebung von Pixeln weg von einem Punkt umfasst, der sich in einem Abschnitt des ersten Anzeigebilds befindet, der einem Punkt innerhalb des mindestens einen blinden Flecks entspricht.

6. Verfahren nach einem der Ansprüche 1 bis 5, wobei das Basissichtfeld mindestens eine Winkelabmessung aufweist, die größer ist als das Gesichtsfeld des ersten Auges; und
die Verschiebung von Pixeln zum Erzeugen des ersten Anzeigebilds derart ist, dass die größere Winkelabmessung der Szene in das Anzeige-Sichtfeld des ersten Anzeigebilds komprimiert wird;
wobei die Verschiebung von Pixeln eine vertikale Verschiebung von Pixeln des ersten Basisbilds in Richtung einer horizontalen Linie in dem ersten Basisbild umfasst, wodurch eine vertikale Winkelabmessung der Szene in das Anzeige-Sichtfeld des ersten Anzeigebilds komprimiert wird, und wobei die vertikale Verschiebung von Pixeln in Richtung der horizontalen Linie inhomogen ist, sodass die Größe der vertikalen Verschiebung umso größer ist, je weiter ein Pixel von der horizontalen Linie entfernt ist.

7. Verfahren nach Anspruch 6, wobei die Größe der vertikalen Verschiebung eines Pixels eine glatte Funktion eines Abstands des Pixels von der horizontalen Linie ist, sodass die vertikale Verschiebung von Pixeln entlang einer vertikalen Linie in dem Basisbild frei von jeglicher Diskontinuität ist.

8. Verfahren nach einem der Ansprüche 1 bis 7, wobei das Basissichtfeld mindestens eine Winkelabmessung aufweist, die größer ist als das Gesichtsfeld des ersten Auges; und
die Verschiebung von Pixeln zum Erzeugen des ersten Anzeigebilds derart ist, dass die größere Winkelabmessung der Szene in das Anzeige-Sichtfeld des ersten Anzeigebilds komprimiert wird;
wobei die Verschiebung von Pixeln eine horizontale Verschiebung von Pixeln des ersten Basisbilds in Richtung einer vertikalen Linie in dem ersten Basisbild umfasst, wodurch eine horizontale Winkelabmessung der Szene in das Anzeige-Sichtfeld des ersten Anzeigebilds komprimiert wird, wobei die horizontale Verschiebung von Pixeln in Richtung der horizontalen Linie inhomogen ist, sodass die Größe der horizontalen Verschiebung umso größer ist, je weiter ein Pixel von der vertikalen Linie entfernt ist.

9. Verfahren nach einem der Ansprüche 1 bis 8, wobei das Basissichtfeld mindestens eine Winkelabmessung aufweist, die größer ist als das Gesichtsfeld des ersten Auges; und
die Verschiebung von Pixeln zum Erzeugen des ersten Anzeigebilds derart ist, dass die größere Winkelabmessung der Szene in das Anzeige-Sichtfeld des ersten Anzeigebilds komprimiert wird;
wobei die Verschiebung von Pixeln eine radiale Verschiebung von Pixeln des ersten Basisbilds in Richtung eines Punkts in dem ersten Basisbild umfasst, wodurch sowohl eine horizontale als auch eine vertikale Winkelabmessung der Szene in das Anzeige-Sichtfeld des ersten Anzeigebilds komprimiert wird, wobei die radiale Verschiebung von Pixeln in Richtung des Punkts inhomogen ist, sodass die Größe der radialen Verschiebung umso größer ist, je weiter ein Pixel von dem Punkt entfernt ist.

10. Verfahren nach einem der Ansprüche 1 bis 9, wobei kein Anzeigebildschirm vor dem zweiten Auge des Menschen positioniert ist.

11. Verfahren nach einem der Ansprüche 1 bis 10, ferner umfassend:
a2. Positionieren eines zweiten Anzeigebildschirms, sodass eine Anzeigefläche des zweiten Anzeigebildschirms im Wesentlichen das gesamte Gesichtsfeld eines zweiten Auges eines Menschen ausfüllt und für das erste Auge des Menschen nicht sichtbar ist;
b2. mit einer zweiten Anzeige-Auffrischungsrate:
i. Extrahieren, aus einem zweiten Videostrom, eines gepixelten, unbewegten zweiten Basisbilds, das ein zweites Basis-Sichtfeld aufweist, wobei das zweite Basisbild eine zweite Szene darstellt und das zweite Basisbild digitale Bilddaten ist, die in einem digitalen Speicher gespeichert sind,
ii. Erzeugen, aus dem zweiten Basisbild, eines gepixelten zweiten Anzeigebilds, das digitale Bilddaten ist, die die gesamte zweite Szene darstellen, wobei das zweite Anzeigebild ein zweites Anzeige-Sichtfeld aufweist, das sich von dem zweiten Basis-Sichtfeld unterscheidet, sodass die zweite Szene, wie sie durch das zweite Anzeigebild dargestellt ist, ein Sichtfeld aufweist, das nicht größer ist als das Gesichtsfeld des zweiten Auges, wobei ein Erzeugen des zweiten Anzeigebilds unter Beibehaltung der Position von jedem Pixel in Bezug auf benachbarte Pixel ein Verschieben von Pixeln umfasst, jedes verschobene Pixel von einer Basiskoordinate in dem zweiten Basisbild zu einer Anzeigekoordinate in dem zweiten Anzeigebild, und
iii. Anzeigen, auf dem zweiten Anzeigebildschirm, des zweiten Anzeigebilds für das zweite Auge, sodass das gesamte zweite Sichtfeld des zweiten Anzeigebilds von dem Gesichtsfeld des zweiten Auges zu einem Zeitpunkt wahrgenommen wird,
wodurch dem zweiten Auge ermöglicht wird, eine Gesamtheit der zweiten Szene zu einem Zeitpunkt wahrzunehmen.

12. Verfahren nach Anspruch 11, wobei die erste Szene und die zweite Szene verschieden sind.

13. Verfahren nach einem der Ansprüche 11 bis 12, wobei ein Abschnitt des ersten Anzeigebilds, der dem gesamten fovealen Gesichtsfeld und einem Teil des makularen Gesichtsfeldes des ersten Auges entspricht, und ein Abschnitt des zweiten Anzeigebilds, der dem gesamten fovealen Gesichtsfeld und einem jeweiligen Teil des makularen Gesichtsfelds des zweiten Auges entspricht, ein binokulares Paar sind, und andere Abschnitte des ersten Anzeigebilds und des zweiten Anzeigebilds keine binokularen Paare sind.

14. Monokulares Headset, das konfiguriert ist, um auf dem Kopf eines Menschen getragen zu werden, umfassend:
einen einzelnen Anzeigebildschirm, der an einem Headset-Körper montiert ist, sodass, wenn der Headset-Körper auf dem Kopf eines Menschen getragen wird, der Anzeigebildschirm positioniert ist, sodass eine Anzeigefläche des Anzeigebildschirms im Wesentlichen das gesamte Gesichtsfeld eines ersten Auges eines Menschen ausfüllt und für ein zweites Auge des Menschen nicht sichtbar ist; und
funktionell assoziiert mit dem Anzeigebildschirm, einen digitalen Prozessor, der einen Videoeingangsanschluss beinhaltet, der konfiguriert ist, um einen Videostrom über den Videoeingangsanschluss zu akzeptieren und das Verfahren nach einem der Ansprüche 1 bis 10 mit einem Videostrom unter Verwendung des Anzeigebildschirms zu implementieren, ferner umfassend einen Eye-Tracker, um die Blickrichtung eines ersten Auges zu bestimmen und die bestimmte Blickrichtung an den Prozessor bereitzustellen.

15. Binokulares Headset, das konfiguriert ist, um auf dem Kopf eines Menschen getragen zu werden, umfassend:
einen ersten Anzeigebildschirm, der an einem Headset-Körper montiert ist, sodass, wenn der Headset-Körper auf dem Kopf eines Menschen getragen wird, der erste Anzeigebildschirm positioniert ist, sodass eine Anzeigefläche des ersten Anzeigebildschirms im Wesentlichen das gesamte Gesichtsfeld eines ersten Auges eines Menschen ausfüllt und für ein zweites Auge des Menschen nicht sichtbar ist;
einen zweiten Anzeigebildschirm, der an dem Headset-Körper montiert ist, sodass, wenn der Headset-Körper auf dem Kopf eines Menschen getragen wird, der zweite Anzeigebildschirm positioniert ist, sodass eine Anzeigefläche des zweiten Anzeigebildschirms im Wesentlichen das gesamte Gesichtsfeld eines zweiten Auges eines Menschen ausfüllt und für das erste Auge des Menschen nicht sichtbar ist; und
funktionell assoziiert mit dem ersten Anzeigebildschirm und mit dem zweiten Anzeigebildschirm einen digitalen Prozessor, der einen Videoeingangsanschluss beinhaltet, der konfiguriert ist, um über den Videoeingangsanschluss einen Videostrom zu akzeptieren und um das Verfahren nach einem der Ansprüche 11 bis 13 mit einem Videostrom unter Verwendung des ersten Anzeigebildschirms und des zweiten Anzeigebildschirms zu implementieren, ferner umfassend einen Eye-Tracker, um die Blickrichtung eines ersten Auges und eines zweiten Auges zu bestimmen und die bestimmten Blickrichtungen an den Prozessor bereitzustellen.

## Revendications

1. Procédé d'affichage d'images comprenant :
a1. le positionnement d'un premier écran d'affichage de sorte qu'une surface d'affichage dudit premier écran d'affichage remplisse sensiblement tout le champ visuel d'un premier œil d'un humain et ne soit pas visible pour un deuxième œil dudit humain ;
b1. à un premier taux de rafraîchissement d'affichage :
i. à partir d'un premier flux vidéo, l'extraction d'une première image de base fixe pixélisée ayant un premier champ de vision de base, ladite première image de base représentant une première scène, ladite première image de base étant des données d'image numérique stockées dans une mémoire numérique ;
ii. à partir de ladite première image de base, la création d'une première image d'affichage pixélisée étant des données d'image numérique représentant ladite première scène entière, ladite première image d'affichage ayant un premier champ de vision d'affichage différent dudit premier champ de vision de base, de sorte que ladite première scène telle que représentée par ladite première image d'affichage ait un champ de vision qui n'est pas plus grand que le champ visuel dudit premier œil, ladite création de ladite première image d'affichage comprenant, tout en conservant la position de chaque pixel par rapport aux pixels voisins, la translation de pixels, chaque pixel translaté depuis une coordonnée de base dans ladite première image de base en une coordonnée d'affichage dans ladite première image d'affichage, et
iii. sur ledit premier écran d'affichage, l'affichage de ladite première image d'affichage audit premier œil de sorte que la totalité dudit premier champ de vision de ladite première image d'affichage soit perçue par le champ visuel dudit premier œil en une seule fois,
permettant ainsi audit premier œil de percevoir la totalité de ladite première scène en une seule fois, dans lequel le champ visuel dudit premier œil comprend au moins un point aveugle et au moins une partie de ladite translation desdits pixels est telle que sensiblement aucun pixel représentant ladite scène ne se trouve au niveau d'une partie de ladite première image d'affichage qui correspond à au moins un desdits au moins un point aveugle, le procédé comprenant en outre :
la détermination d'une direction de regard dudit premier oeil ; et
l'affichage et/ou la création de ladite première image d'affichage également conformément à ladite direction de regard déterminée de sorte que sensiblement aucun pixel représentant ladite scène ne se trouve au niveau d'une partie de ladite première image d'affichage qui correspond à au moins l'un desdits au moins un point aveugle dans le champ visuel dudit premier œil.

2. Procédé selon la revendication 1, dans lequel pendant ladite création de ladite première image d'affichage, les pixels d'une partie spécifique de ladite première image de base sont préservés et non translatés de sorte que la partie préservée de ladite première image d'affichage correspondant à ladite partie préservée de ladite première image de base soit la même.

3. Procédé selon l'une quelconque des revendications 1 à 2, dans lequel pendant ladite création de ladite première image d'affichage, les pixels des parties de ladite première image de base correspondant à au moins une partie du champ visuel fovéal dudit premier œil ne sont pas translatés de sorte que lesdites parties de ladite première image d'affichage correspondant à ladite partie du champ visuel fovéal dudit premier œil soient les mêmes que la partie correspondante de ladite première image de base.

4. Procédé selon l'une quelconque des revendications 1 à 3, dans lequel pendant ladite création de ladite première image d'affichage, les pixels des parties de ladite première image de base correspondant au champ visuel maculaire dudit premier œil ne sont pas translatés de sorte que les parties de ladite première image d'affichage correspondant au champ visuel maculaire dudit premier œil soient les mêmes que la partie correspondante de ladite première image de base.

5. Procédé selon l'une quelconque des revendications 1 à 4,
dans lequel ladite translation de pixels pendant ladite création de ladite première image d'affichage comprend une translation radiale vers l'extérieur de pixels à distance d'un point situé au niveau d'une partie de ladite première image d'affichage qui correspond à un point à l'intérieur dudit au moins un point aveugle.

6. Procédé selon l'une quelconque des revendications 1 à 5, dans lequel ledit champ de vision de base a au moins une dimension angulaire plus grande que le champ visuel dudit premier œil ; et
ladite translation de pixels pour ladite création de ladite première image d'affichage est telle que ladite dimension angulaire plus grande de ladite scène est compressée dans ledit champ de vision d'affichage de ladite première image d'affichage ;
dans lequel ladite translation de pixels comprend la translation verticale de pixels de ladite première image de base vers une ligne horizontale dans ladite première image de base, comprimant ainsi une dimension angulaire verticale de ladite scène dans ledit champ de vision d'affichage de ladite première image d'affichage, et
dans lequel ladite translation verticale de pixels vers ladite ligne horizontale est inhomogène de sorte que plus un pixel est éloigné de ladite ligne horizontale, plus l'amplitude de ladite translation verticale soit grande.

7. Procédé selon la revendication 6, dans lequel l'amplitude de la translation verticale d'un pixel est une fonction lissée d'une distance dudit pixel depuis ladite ligne horizontale de sorte que ladite translation verticale des pixels le long d'une ligne verticale dans ladite image de base soit dépourvue de toute discontinuité.

8. Procédé selon l'une quelconque des revendications 1 à 7, dans lequel ledit champ de vision de base a au moins une dimension angulaire plus grande que le champ visuel dudit premier œil ; et
ladite translation de pixels pour ladite création de ladite première image d'affichage est telle que ladite dimension angulaire plus grande de ladite scène est comprimée dans ledit champ de vision d'affichage de ladite première image d'affichage ;
dans lequel ladite translation de pixels comprend la translation horizontale de pixels de ladite première image de base vers une ligne verticale dans ladite première image de base, comprimant ainsi une dimension angulaire horizontale de ladite scène dans ledit champ de vision d'affichage de ladite première image d'affichage, dans lequel ladite translation horizontale de pixels vers ladite ligne verticale est inhomogène de sorte que plus un pixel est éloigné de ladite ligne verticale, plus l'amplitude de ladite translation horizontale soit grande.

9. Procédé selon l'une quelconque des revendications 1 à 8, dans lequel ledit champ de vision de base a au moins une dimension angulaire plus grande que le champ visuel dudit premier œil ; et
ladite translation de pixels pour ladite création de ladite première image d'affichage est telle que ladite dimension angulaire plus grande de ladite scène est comprimée dans ledit champ de vision d'affichage de ladite première image d'affichage ;
dans lequel ladite translation de pixels comprend la translation radiale de pixels de ladite première image de base vers un point dans ladite première image de base, comprimant ainsi à la fois une dimension angulaire horizontale et verticale de ladite scène dans ledit champ de vision d'affichage de ladite première image d'affichage, dans lequel ladite la translation radiale des pixels vers ledit point est inhomogène de sorte que plus un pixel est éloigné dudit point, plus l'amplitude de ladite translation radiale soit grande.

10. Procédé selon l'une quelconque des revendications 1 à 9, dans lequel aucun écran d'affichage n'est positionné devant ledit deuxième œil dudit humain.

11. Procédé selon l'une quelconque des revendications 1 à 10, comprenant en outre :
a2. le positionnement d'un deuxième écran d'affichage de sorte qu'une surface d'affichage dudit deuxième écran d'affichage remplisse sensiblement tout le champ visuel d'un deuxième œil d'un humain et ne soit pas visible pour ledit premier œil dudit humain ;
b2. à un deuxième taux de rafraîchissement d'affichage :
i. à partir d'un deuxième flux vidéo, l'extraction d'une deuxième image de base fixe pixélisée ayant un deuxième champ de vision de base, ladite deuxième image de base représentant une deuxième scène, ladite deuxième image de base étant des données d'image numérique stockées dans une mémoire numérique,
ii. à partir de ladite deuxième image, la création d'une deuxième image d'affichage pixélisée étant des données d'image numérique représentant ladite deuxième scène entière, ladite deuxième image d'affichage ayant un deuxième champ de vision d'affichage différent dudit deuxième champ de vision de base, de sorte que ladite deuxième scène telle que représentée par ladite deuxième image d'affichage ait un champ de vision qui n'est pas plus grand que le champ visuel dudit deuxième œil, ladite création de ladite deuxième image d'affichage comprenant, tout en conservant la position de chaque pixel par rapport aux pixels voisins, la translation de pixels, chaque pixel translaté depuis une coordonnée de base dans ladite deuxième image de base en une coordonnée d'affichage dans ladite deuxième image d'affichage, et
iii. sur ledit deuxième écran d'affichage, l'affichage de ladite deuxième image d'affichage audit deuxième œil de sorte que la totalité dudit deuxième champ de vision de ladite deuxième image d'affichage soit perçue par le champ visuel dudit deuxième œil en une seule fois,
permettant ainsi audit deuxième œil de percevoir la totalité de ladite deuxième scène en une seule fois.

12. Procédé selon la revendication 11, dans lequel ladite première scène et ladite deuxième scène sont différentes.

13. Procédé selon l'une quelconque des revendications 11 à 12, dans lequel une partie de ladite première image d'affichage qui correspond à la totalité du champ visuel fovéal et une partie du champ visuel maculaire dudit premier œil et une partie de ladite deuxième image d'affichage qui correspond à la totalité du champ visuel fovéal et une partie respective du champ visuel maculaire dudit deuxième œil sont une paire binoculaire, et d'autres parties de ladite première image d'affichage et de ladite deuxième image d'affichage ne sont pas des paires binoculaires.

14. Casque monoculaire configuré pour être porté sur la tête d'un humain, comprenant :
un écran d'affichage unique monté sur un corps de casque, de sorte que lorsque ledit corps de casque est porté sur la tête d'un humain, ledit écran d'affichage soit positionné de sorte qu'une surface d'affichage dudit écran d'affichage remplisse sensiblement tout le champ visuel d'un premier œil d'un humain et ne soit pas visible pour un deuxième œil dudit humain ; et
fonctionnellement associé audit écran d'affichage, un processeur numérique comprenant un port d'entrée vidéo configuré pour accepter un flux vidéo via ledit port d'entrée vidéo et pour mettre en œuvre le procédé de l'une quelconque des revendications 1 à 10 avec un dit flux vidéo en utilisant ledit écran d'affichage, en outre comprenant un oculomètre pour déterminer la direction du regard d'un dit premier œil et pour fournir la direction du regard déterminée audit processeur.

15. Casque binoculaire configuré pour être porté sur la tête d'un humain, comprenant :
un premier écran d'affichage monté sur un corps de casque, de sorte que lorsque ledit corps de casque est porté sur la tête d'un humain, ledit premier écran d'affichage soit positionné de sorte qu'une surface d'affichage dudit premier écran d'affichage remplisse sensiblement tout le champ visuel d'un premier l'œil d'un humain et ne soit pas visible pour un deuxième œil dudit humain ;
un deuxième écran d'affichage monté sur ledit corps de casque, de sorte que lorsque ledit corps de casque est porté sur la tête d'un humain, ledit deuxième écran d'affichage soit positionné de sorte qu'une surface d'affichage dudit deuxième écran d'affichage remplisse sensiblement tout le champ visuel d'un deuxième l'œil d'un humain et ne soit pas visible pour ledit premier œil dudit humain ; et
fonctionnellement associé audit premier écran d'affichage et audit deuxième écran d'affichage, un processeur numérique comprenant un port d'entrée vidéo configuré pour accepter un flux vidéo via ledit port d'entrée vidéo et pour mettre en œuvre le procédé de l'une quelconque des revendications 11 à 13 avec un dit flux vidéo utilisant ledit premier écran d'affichage et ledit deuxième écran d'affichage, comprenant en outre un oculomètre pour déterminer la direction du regard dudit premier œil et dudit deuxième œil, et pour fournir les directions du regard déterminées audit processeur.
